# EUROPEAN PATENT SPECIFICATION

(11) **EP 4 519 384 B1**
(45) Date of publication and mention of the grant of the patent: **29.04.2026**
(21) Application number: 23728186.0
(22) Date of filing: 04.05.2023
(51) Int. Cl.: C09K 11/06, G01N 33/542, G01N 33/58

(54) **PHOTOACTIVATABLE COMPOUNDS AND USES THEREOF**
PHOTOAKTIVIERBARE VERBINDUNGEN UND VERWENDUNGEN DAVON
COMPOSÉS PHOTOACTIVABLES ET LEURS UTILISATIONS

(30) Priority: 04.05.2022 US 202263338322 P
(43) Date of publication of application: 12.03.2025
(73) Proprietor: Promega Corporation, Madison, WI 53711 (US)
(72) Inventor: LARSEN, Matthew A., Madison, Wisconsin 53711 (US); OHANA, Rachel Friedman, Madison, Wisconsin 53711 (US); ZHOU, Wenhui, Madison, Wisconsin 53711 (US); SHI, Ce, Madison, Wisconsin 53711 (US); CAO, Jian, Madison, Wisconsin 53711 (US); HURST, Robin, Madison, Wisconsin 53711 (US); KLEIN, Mark A., Madison, Wisconsin 53711 (US); WANG, Hui, Madison, Wisconsin 53711 (US); AN, Weiwei, Madison, Wisconsin 53711 (US); PORTER, Karilyn, Madison, Wisconsin 53711 (US); MACHLEIDT, Thomas, Madison, Wisconsin 53711 (US)
(74) Representative: Potter Clarkson
(86) International application number: PCT/US2023/021030
(87) International publication number: WO 2023/215497

(56) References cited:
- WO-A2-02/056013

## Description

### CROSS-REFERENCE TO RELATED APPLICATIONS

This application claims priority to and the benefit of U.S. Provisional Patent Application No. 63/338,322, filed on May 4, 2022.

### FIELD

The present invention relates to compounds, compositions, systems, and methods for photoactivatable labeling, which can be actuated within biological systems. In particular, compounds disclosed herein include vinyl-extended-aryl azide moieties that undergo photoactivation to generate reactive intermediates, which can form covalent linkages with biomolecules. Photoactivation can be conducted by a variety of mechanisms including ultraviolet (UV) irradiation, visible light irradiation, or energy transfer (e.g., from a photocatalyst). The compounds also include functional moieties that provide useful functionalities, for example detection and /or enrichment of biomolecules, such as fluorophores, capture elements (e.g., biotin), reactive moieties (e.g., click handles), and bifunctional moieties (e.g., a moiety comprising a bioactive compound and either a fluorophore or a capture element or a reactive moiety).

### BACKGROUND

The need to study and map dynamic microenvironments, molecular networks, and small molecules interactions as well as protein-protein and protein-nucleic acid interactions in physiologically relevant contexts create a demand for new functional biological tools to enable such analyses in live cells and complex models in a nondestructive fashion.
WO 02/056013 A2 relates to oxazolidinone photoaffinity probes, uses and compounds.

### SUMMARY

The invention is as set out in the appended set of claims. In accordance with the invention, one aspect disclosed herein is a compound of formula (I): 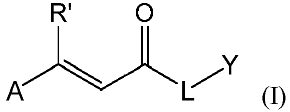 or a salt thereof, wherein:
A is selected from: 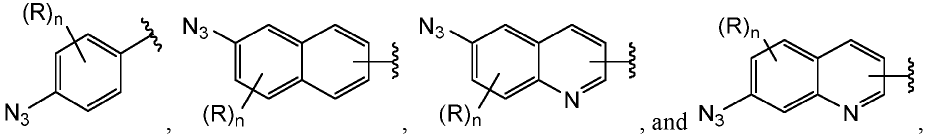 wherein:
   each n is independently 1, 2, 3, or 4; and
   each R is independently selected from hydrogen, halo, C₁-C₄ alkyl, C₂-C₄ alkenyl, hydroxy, mercapto, amino, cyano, C₁-C₄-alkoxy, halo-C₁-C₄-alkyl, hydroxy-C₁-C₄-alkyl, amino-C₁-C₄-alkyl, mercapto-C₁-C₄-alkyl, cyano-C₁-C₄-alkyl, - C(O)-C₁-C₄-alkyl, -C(O)OH, and -C(O)NH₂;
R' is hydrogen or C₁-C₄ alkyl;
L is a linker; and
Y is a functional moiety selected from:
   (a) biotin;
   (b) a haloalkane group;
   (c) a fluorescent functional group selected from a xanthene, a cyanine, a naphthalene, an oxadiazole, a pyrene, an oxazine, an acridine, an arylmethine, a tetrapyrrole, a coumarin, a squaraine, and a boron-dipyrromethene; and
   (d) a reactive functional group comprising an azide, alkyne, alkene, or 1,2,4,5-tetrazinyl moiety.

In some embodiments, A is: 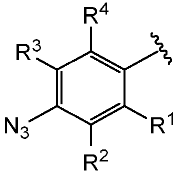 wherein:
R¹, R², R³, and R⁴ are each independently selected from hydrogen, halo, hydroxy, cyano, and C₁-C₄ alkoxy.

In some embodiments: R¹ is hydrogen, hydroxy, or C₁-C₄ alkoxy; R² is hydrogen, halo, cyano, or C₁-C₄ alkoxy; R³ is hydrogen or halo; and R⁴ is hydrogen or halo.

In some embodiments, A is: 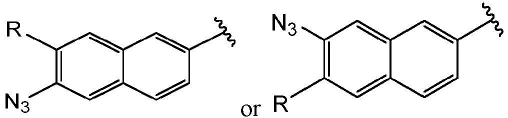
wherein each R is independently selected from hydrogen, halo, cyano, and C₁-C₄ alkoxy. In some embodiments, A is: 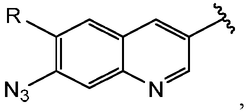
wherein R is selected from hydrogen, halo, cyano, and C₁-C₄ alkoxy. In some embodiments, A is: 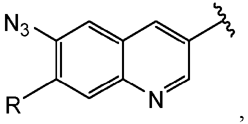
wherein R is selected from hydrogen, halo, cyano, and C₁-C₄ alkoxy. In some embodiments, A has a formula selected from: 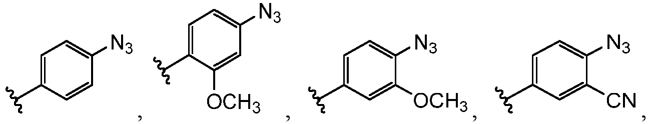 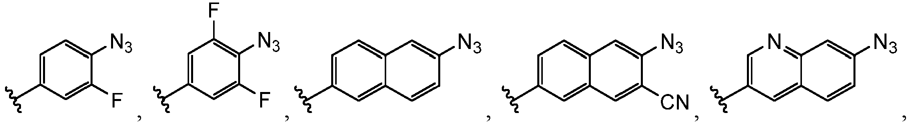 and

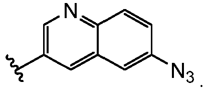

In some embodiments, R' is selected from hydrogen and methyl.

In some embodiments, the linker comprises one or more moieties selected from straight or branched chain alkylene, ether (-O-), amine (-NH-), ester (-C(O)O-), amide (-C(O)NH-), carbamate (-NHC(O)O-), urea (-NHC(O)NH-), and phenylene groups.

In some embodiments, the linker has a formula:

-NHCH₂CH₂(OCH₂CH₂)ₙNH-

wherein n is 1, 2, 3, 4, 5, 6, 7, or 8.

In some embodiments otherwise disclosed herein, Y is a functional moiety selected from a capture element, a detectable moiety, a reactive moiety, and a bifunctional moiety.

In some embodiments, Y is a capture element selected from biotin and a haloalkane group. In some embodiments, Y has a formula: 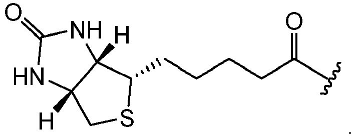 In some embodiments, Y has a formula -(CH₂)ₙ-X, wherein n is 2, 3, 4, 5, 6, 7, 8, 9, 10, 11, or 12, and X is a halogen.

In some embodiments otherwise disclosed herein, Y is a detectable moiety. In some embodiments, Y is a fluorescent functional group. In some embodiments, Y is a fluorescent functional group selected from selected from a xanthene, a cyanine, a naphthalene, an oxadiazole, a pyrene, an oxazine, an acridine, an arylmethine, a tetrapyrrole, a coumarin, a squaraine, and a boron-dipyrromethene. In some embodiments, Y is a fluorogenic functional group.

In some embodiments, Y is a reactive functional group. In some embodiments, Y is a reactive functional group comprising an azide, alkyne, alkene, or 1,2,4,5-tetrazinyl moiety.

In some embodiments otherwise disclosed herein, Y is a bifunctional moiety comprising: (i) a bioactive compound; and (ii) a capture element or a fluorescent moiety or a reactive moiety.

In some embodiments, the compound of formula (I) is a compound selected from the group consisting of: 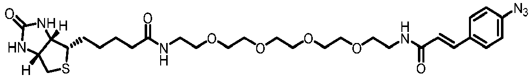 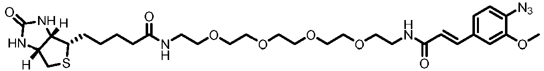 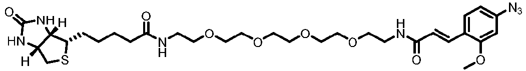 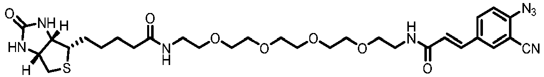 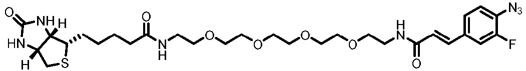 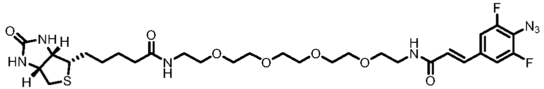 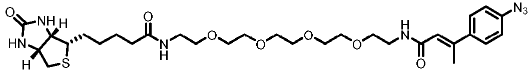 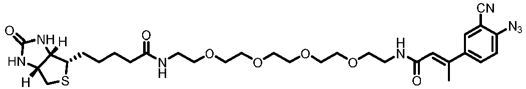 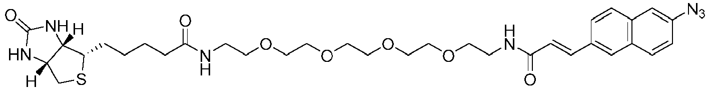 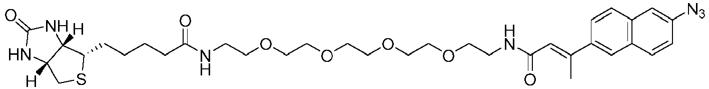 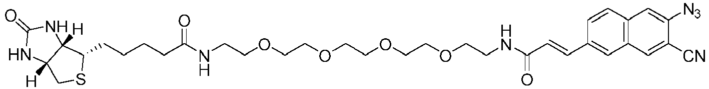 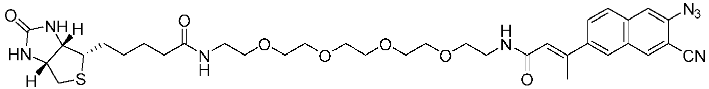 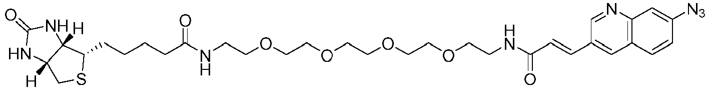 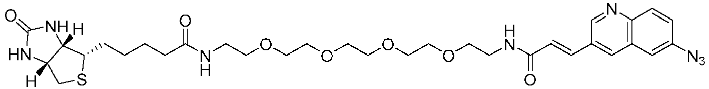 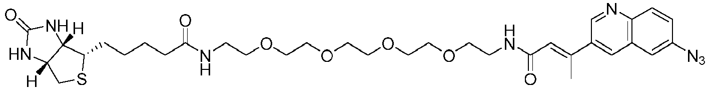 and salts thereof.

In accordance with the invention, another aspect disclosed herein is a system for photocatalytic labeling of a biomolecule, comprising:
(a) a compound of formula (I) (e.g., any compound of formula (I) disclosed herein); and
(b) a photocatalyst.

In some embodiments, the photocatalyst has a structure 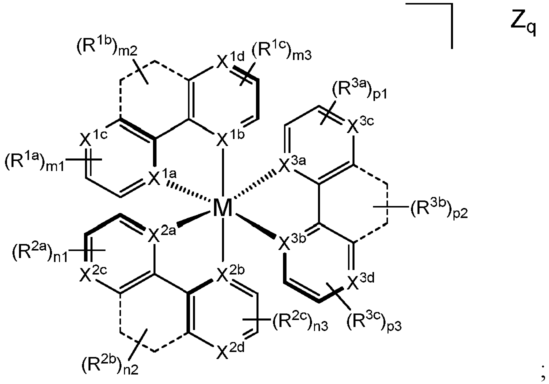 wherein:
each set of dashed lines (------) represents the presence or absence of a fused 6-membered ring;
M is a transition metal;
m1, m2, m3, n1, n2, n3, p1, p2, and p3 are each independently 0, 1, or 2;
R^{1a}, R^{1b}, R^{1c}, R^{2a}, R^{2b}, R^{2c}, R^{3a}, R^{3b}, and R^{3c} are each independently selected from halo, alkyl, haloalkyl, amino, and heteroalkyl;
X^{1a}, X^{1b}, x^{2a}, X^{2b}, X^{3a}, and X^{3b} are each independently selected from N and C, wherein at least one of X^{1a} and X^{1b} is N, at least one of X^{2a} and X^{2b} is N, and at least one of X^{3a} and X^{3b} is N;
X^{1c}, X^{1d}, X^{2c}, X^{2d}, X^{3c}, and X^{3d} are each independently selected from CH and N;
Z is an anion; and
q is 0, 1, or 2.

In some embodiments, the transition metal is selected from Ru and Ir.

In some embodiments, the photocatalyst is an iridium photocatalyst selected from:

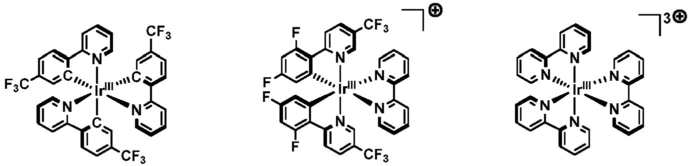

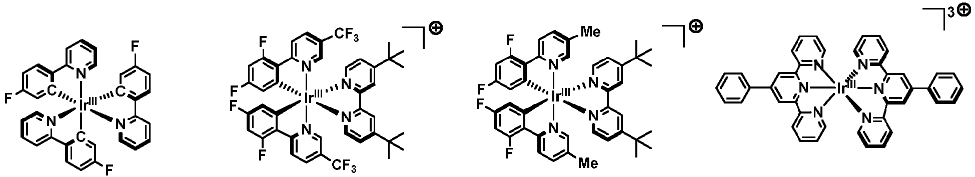

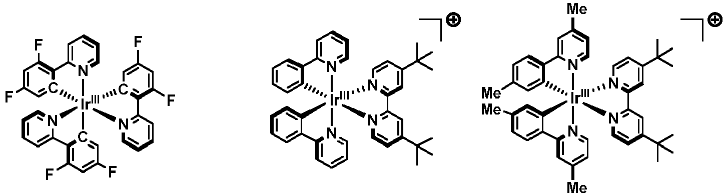

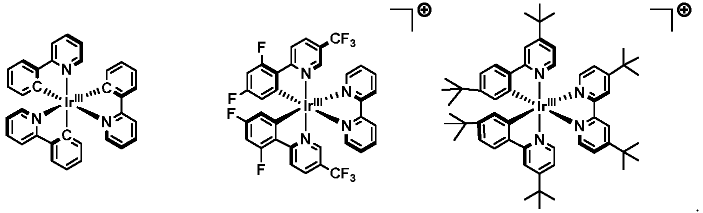

In some embodiments, the photocatalyst is a ruthenium-based photocatalyst selected from:

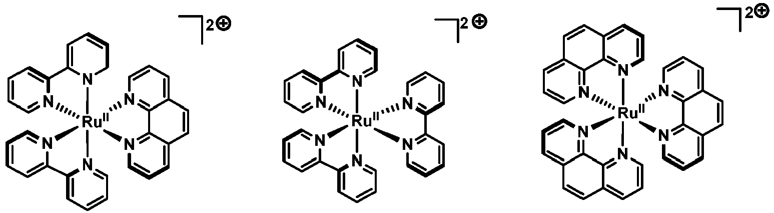

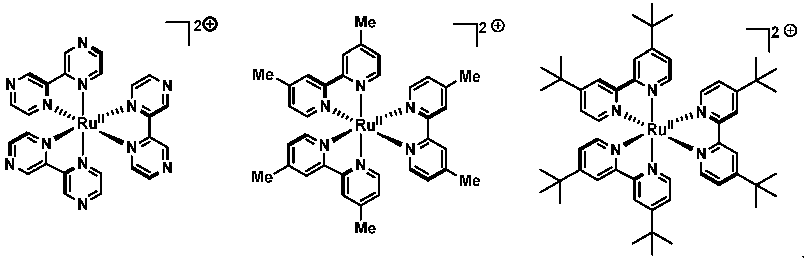

In some embodiments, the photocatalyst is of the formula:

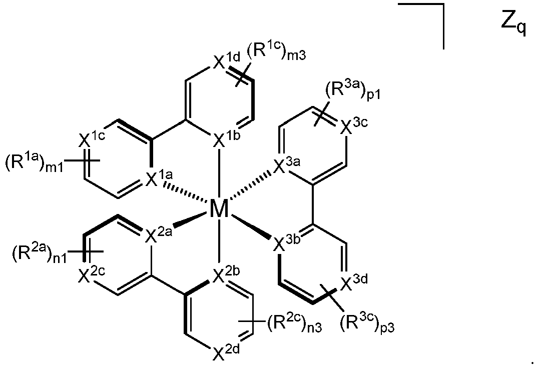

In accordance with the invention, one aspect disclosed herein is a method of labeling a biomolecule in a sample, comprising:
(a) contacting the sample with a compound of formula (I) (e.g., any compound of formula (I) disclosed herein); and
(b) exposing the sample to light.

In some embodiments, the light is selected from ultraviolet light and visible light. In some embodiments, the light is visible light from a light-emitting diode. In some embodiments, the light is bioluminescent light. In some embodiments, the method further comprises contacting the sample with a photocatalyst in step (a).

### BRIEF DESCRIPTION OF THE DRAWINGS

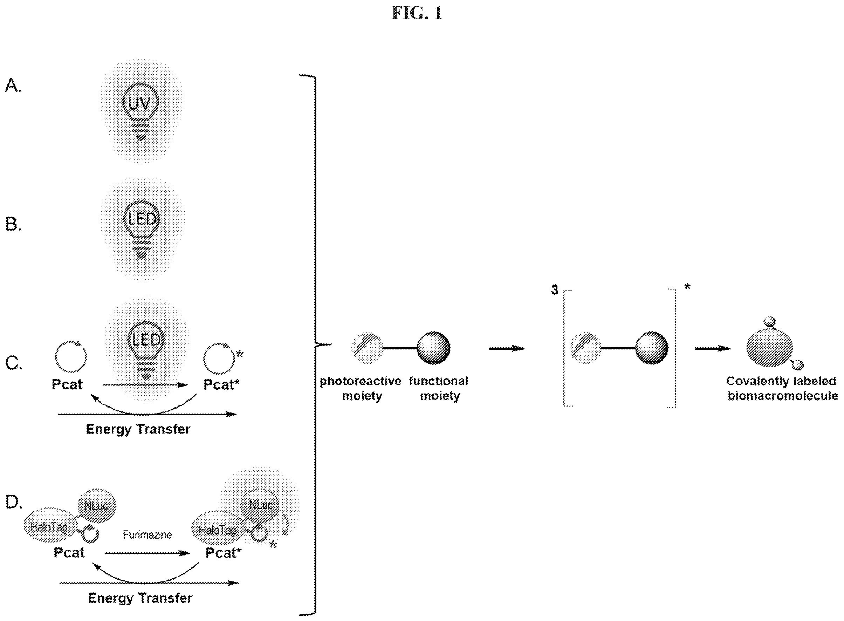

FIG. 1 shows a cartoon depiction of covalent labeling using a probe comprising a vinyl-extended-aryl-azide-based photoreactive group, which upon activation generates a short-lived reactive intermediate that is capable of forming a covalent linkage with neighboring biomolecules. Activation modes includes: (A) UV irradiation, (B) Visible 455 nm LED irradiation, (C) LED-triggered activation of a light sensitive catalyst, which further engages in energy transfer events with the vinyl-extended-aryl-azide-based photoreactive group, and (D) bioluminescence-triggered activation of a light sensitive catalyst, which further engages in energy transfer events with the vinyl-extended-aryl-azide-based photoreactive group.

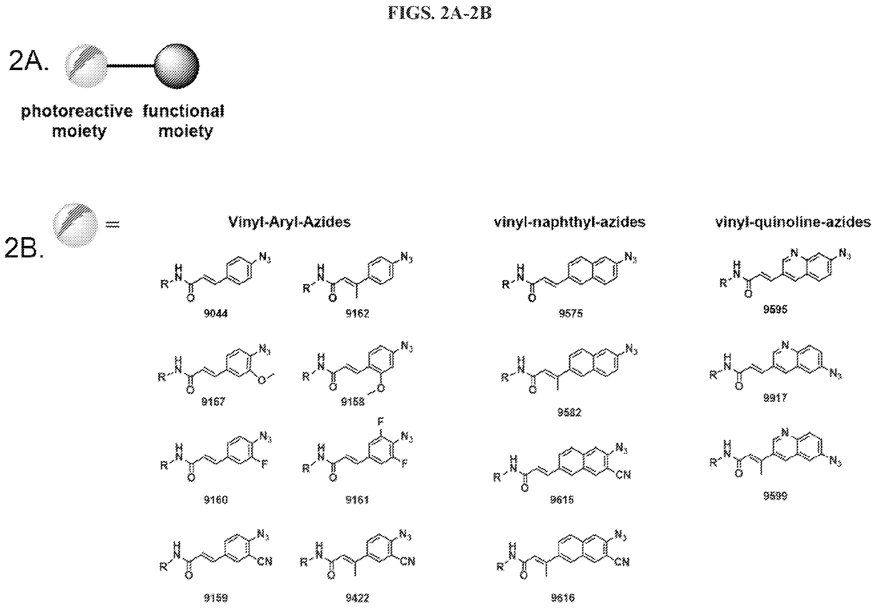

FIGS. 2A-2B show: (2A) a schematic representation of a probe comprising a vinyl-extended-aryl-azide-based photoreactive group linked to a functional moiety via a linker; (2B) structures of the vinyl-extended-aryl-azide-based photoreactive groups.

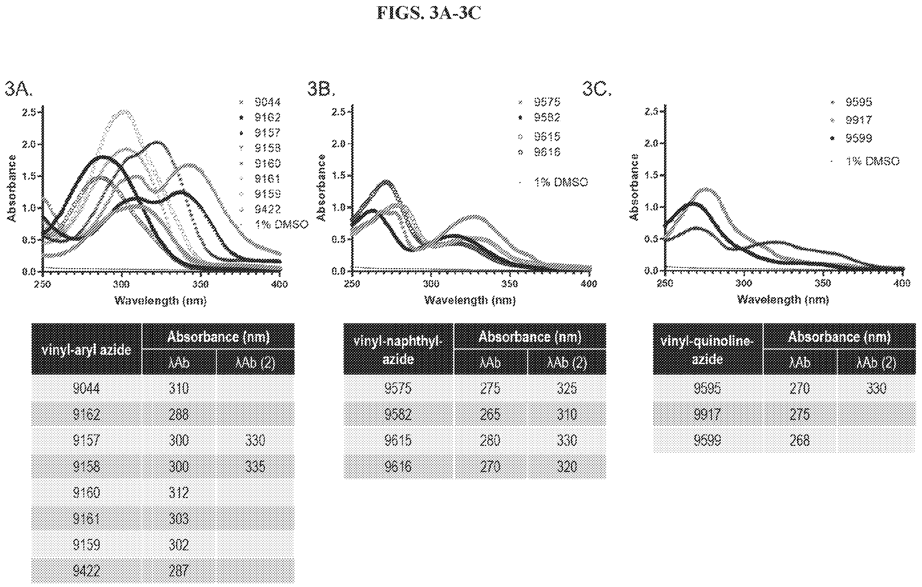

FIGS. 3A-3C show absorbance profiles for a range of vinyl-extended-aryl-azide-based photoreactive groups.

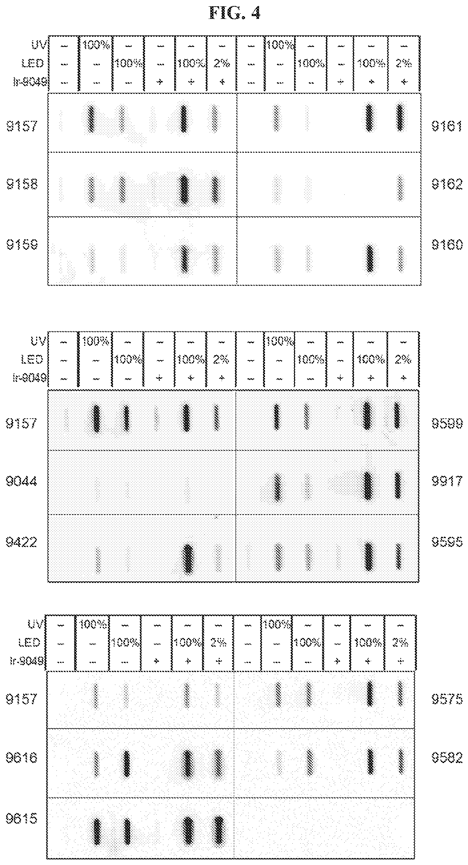

FIG. 4 shows data for evaluation of crosslinking efficiencies for a range of vinyl-extended-aryl-azide-based photoreactive groups; in particular, the data show slot blot analyses of covalent protein labeling induced by either direct irradiation with UV or visible light (455 nm LED) or LED-triggered activation of an iridium catalyst, which further engages in energy transfer events with the vinyl-extended-aryl-azide-based photoreactive group.

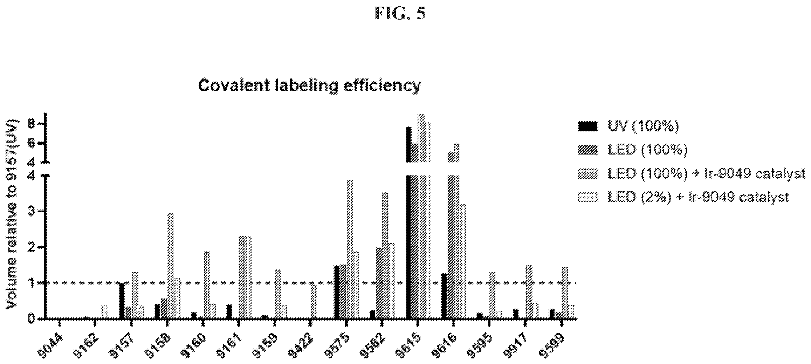

FIG. 5 shows data evaluating covalent labeling efficiencies for a range of vinyl-extended aryl-azide-based photoreactive groups; in particular, the data shows quantitation of the slot blot analyses from FIG. 4.

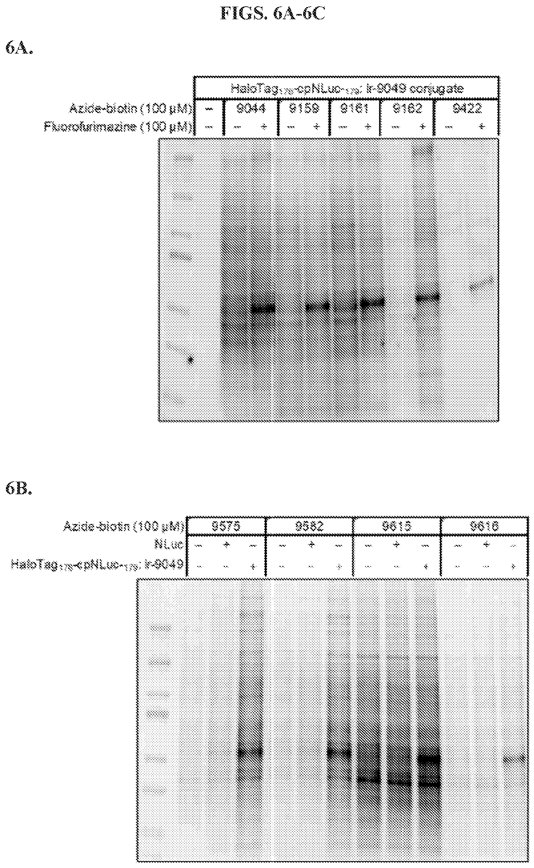

FIGS. 6A-6C show data for evaluation of a range of vinyl-extended-aryl-azide-based photoreactive groups for their capacity to undergo bioluminescence-triggered photocatalytic covalent protein labeling; in particular, the data show Western analyses of covalent protein labeling induced by bioluminescence-triggered activation of an iridium catalyst, which further engages in energy transfer events with the vinyl-extended-aryl-azide-based photoreactive group.

### DEFINITIONS

Although any methods and materials similar or equivalent to those described herein can be used in the practice or testing of embodiments described herein, some preferred methods, compositions, devices, and materials are described herein. However, before the present materials and methods are described, it is to be understood that this invention is not limited to the particular molecules, compositions, methodologies, or protocols herein described, as these may vary in accordance with routine experimentation and optimization. It is also to be understood that the terminology used in the description is for the purpose of describing the particular versions or embodiments only, and is not intended to limit the scope of the embodiments described herein.

Unless otherwise defined, all technical and scientific terms used herein have the same meaning as commonly understood by one of ordinary skill in the art to which this invention belongs. However, in case of conflict, the present specification, including definitions, will control. Accordingly, in the context of the embodiments described herein, the following definitions apply.

As used herein and in the appended claims, the singular forms "a," "an," and "the" include plural reference unless the context clearly dictates otherwise. Thus, for example, reference to "a peptide" is a reference to one or more peptides and equivalents thereof known to those skilled in the art, and so forth.

As used herein, the term "and/or" includes any and all combinations of listed items, including any of the listed items individually. For example, "A, B, and/or C" encompasses A, B, C, AB, AC, BC, and ABC, each of which is to be considered separately described by the statement "A, B, and/or C."

As used herein, the term "comprise" and linguistic variations thereof denote the presence of recited feature(s), element(s), method step(s), etc., without the exclusion of the presence of additional feature(s), element(s), method step(s), etc. Conversely, the term "consisting of" and linguistic variations thereof, denotes the presence of recited feature(s), element(s), method step(s), etc., and excludes any unrecited feature(s), element(s), method step(s), etc., except for ordinarily-associated impurities. The phrase "consisting essentially of" denotes the recited feature(s), element(s), method step(s), etc. and any additional feature(s), element(s), method step(s), etc., that do not materially affect the basic nature of the composition, system, or method. Many embodiments herein are described using open "comprising" language. Such embodiments encompass multiple closed "consisting of" and/or "consisting essentially of" embodiments, which may alternatively be claimed or described using such language.

As used herein, the term "substantially" means that the recited characteristic, parameter, and/or value need not be achieved exactly, but that deviations or variations, including for example, tolerances, measurement error, measurement accuracy limitations and other factors known to skill in the art, may occur in amounts that do not preclude the effect the characteristic was intended to provide. A characteristic or feature that is substantially absent (e.g., substantially non-luminescent) may be one that is within the noise, beneath background, below the detection capabilities of the assay being used, or a small fraction (e.g., <1%, <0.1%, <0.01%, <0.001%, <0.00001%, <0.000001%, <0.0000001%) of the significant characteristic (e.g., luminescent intensity of a bioluminescent protein or bioluminescent complex).

As used herein, the term "biomolecule" or "biological molecule" refers to molecules and ions that are present in organisms and are essential to a biological process(es) such as cell division, morphogenesis, or development. Biomolecules include large macromolecules (or polyanions) such as proteins, carbohydrates, lipids, and nucleic acids as well as small molecules such as primary metabolites, secondary metabolites, and natural products. A more general name for this class of material is biological materials. Biomolecules are usually endogenous, but may also be exogenous. For example, pharmaceutical drugs may be natural products or semisynthetic (biopharmaceuticals), or they may be totally synthetic.

Definitions of specific functional groups and chemical terms are described in more detail below. For purposes of this disclosure, the chemical elements are identified in accordance with the Periodic Table of the Elements, CAS version, Handbook of Chemistry and Physics, 75^{th} Ed., inside cover, and specific functional groups are generally defined as described therein. Additionally, general principles of organic chemistry, as well as specific functional moieties and reactivity, are described in Sorrell, Organic Chemistry, 2nd edition, University Science Books, Sausalito, 2006; Smith, March's Advanced Organic Chemistry: Reactions, Mechanism, and Structure, 7th Edition, John Wiley & Sons, Inc., New York, 2013; Larock, Comprehensive Organic Transformations, 3rd Edition, John Wiley & Sons, Inc., New York, 2018; and Carruthers, Some Modern Methods of Organic Synthesis, 3rd Edition, Cambridge University Press, Cambridge, 1987.

As used herein, the term "alkyl" means a straight or branched saturated hydrocarbon chain containing from 1 to 30 carbon atoms, for example 1 to 16 carbon atoms (C₁-C₁₆ alkyl), 1 to 14 carbon atoms (C₁-C₁₄ alkyl), 1 to 12 carbon atoms (C₁-C₁₂ alkyl), 1 to 10 carbon atoms (C₁-C₁₀ alkyl), 1 to 8 carbon atoms (C₁-C₈ alkyl), 1 to 6 carbon atoms (C₁-C₆ alkyl), 1 to 4 carbon atoms (C₁-C₄ alkyl), 6 to 20 carbon atoms (C₆-C₂₀ alkyl), or 8 to 14 carbon atoms (C₈-C₁₄ alkyl). Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl, n-undecyl, and n-dodecyl.

As used herein, the term "alkenyl" means a straight or branched hydrocarbon chain containing at least one carbon-carbon double bond. The double bond(s) may be located at any positions with the hydrocarbon chain. Representative examples of alkenyl include, but are not limited to, ethenyl, 2-propenyl, 2-methyl-2-propenyl, 3-butenyl, 4-pentenyl, 5-hexenyl, 2-heptenyl, 2-methyl-1-heptenyl, and 3-decenyl.

As used herein, the term "alkynyl" means a straight or branched hydrocarbon chain containing at least one carbon-carbon triple bond. The triple bond(s) may be located at any position within the hydrocarbon chain. Representative examples of alkynyl include, but are not limited to, ethynyl, propynyl, and butynyl.

As used herein, the term "alkylene" means a divalent alkyl radical (e.g., -CH₂CH₂-). As used herein, the term "alkenylene" means a divalent alkenyl radical (e.g., -CH₌CH-). As used herein, the term "alkynylene" means a divalent alkynyl radical (e.g., -C≡C-).

As used herein, the term "alkoxy" refers to an alkyl group, as defined herein, appended to the parent molecular moiety through an oxygen atom. Representative examples of alkoxy include, but are not limited to, methoxy, ethoxy, propoxy, 2-propoxy, butoxy, and tert-butoxy.

As used herein, the term "amino" means a -NH₂ group.

As used herein, the term "aminoalkyl" means an alkyl group, as defined herein, in which at least one hydrogen atom is replaced with an amino group, as defined herein. Representative examples of aminoalkyl include, but are not limited to, aminomethyl, 2-aminoethyl, 2-aminopropyl, 3-aminopropyl, and 4-aminobutyl.

As used herein, the term "cyano" means a -CN group.

As used herein, the term "cyanoalkyl" means an alkyl group, as defined herein, in which at least one hydrogen atom is replaced with a cyano group, as defined herein. Representative examples of cyanoalkyl include, but are not limited to, cyanomethyl, 2-cyanoethyl, 2-cyanopropyl, 3-cyanopropyl, and 4-cyanobutyl.

As used herein, the term "halogen" or "halo" means F, Cl, Br, or I.

As used herein, the term "haloalkyl" means an alkyl group, as defined herein, in which at least one hydrogen atom (e.g., one, two, three, four, five, six, seven or eight hydrogen atoms) is replaced with a halogen. In some embodiments, each hydrogen atom of the alkyl group is replaced with a halogen. Representative examples of haloalkyl include, but are not limited to, fluoromethyl, difluoromethyl, trifluoromethyl, 2,2,2-trifluoroethyl, and 3,3,3-trifluoropropyl.

As used herein, the term "heteroalkyl" means an alkyl group, as defined herein, in which one or more of the carbon atoms (and any associated hydrogen atoms) are each independently replaced with a heteroatom group such as -NR-, -O-, -S-, -S(O)-, -S(O)₂-, and the like, where R is H, alkyl, aryl, cycloalkyl, heteroalkyl, heteroaryl, or heterocyclyl, each of which may be optionally substituted. By way of example, 1, 2, or 3 carbon atoms may be independently replaced with the same or different heteroatomic group. Examples of heteroalkyl groups include, but are not limited to, -OCH₃, -CH₂OCH₃, -SCH₃, -CH₂SCH₃, -NRCH₃, and -CH₂NRCH₃, where R is hydrogen, alkyl, aryl, arylalkyl, heteroalkyl, or heteroaryl, each of which may be optionally substituted. Heteroalkyl also includes groups in which a carbon atom of the alkyl is oxidized (i.e., is -C(O)-).

As used herein, the term "hydroxy" means a -OH group.

As used herein, the term "hydroxyalkyl" means an alkyl group, as defined herein, in which at least one hydrogen atom is replaced with a hydroxy group. Representative examples of hydroxyalkyl include, but are not limited to, hydroxymethyl, 2-hydroxyethyl, 2-hydroxypropyl, 3-hydroxypropyl, and 4-hydroxybutyl.

As used herein, the term "mercapto" means a -SH group.

As used herein, the term "mercaptoalkyl" means an alkyl group, as defined herein, in which at least one hydrogen atom is replaced with a mercapto group. Representative examples of mercaptoalkyl include, but are not limited to, mercaptomethyl, 2-mercaptoethyl, 2-mercaptopropyl, 3-mercaptopropyl, and 4-mercaptobutyl.

As used herein, in chemical structures the indication: 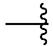 represents a point of attachment of one moiety to another moiety (e.g., a substituent group to the rest of the compound).

For compounds described herein, groups and substituents thereof may be selected in accordance with permitted valence of the atoms and the substituents, such that the selections and substitutions result in a stable compound, e.g., which does not spontaneously undergo transformation such as by rearrangement, cyclization, elimination, etc.

When substituent groups are specified by their conventional chemical formula, written from left to right, such indication also encompass substituent groups resulting from writing the structure from right to left. For example, if a bivalent group is shown as -CH₂O-, such indication also encompasses -OCH₂-; similarly, -OC(O)NH- also encompasses -NHC(O)O-. When linker moieties are shown, the linkers can be attached to other moieties of the compound in either direction.

As used herein, the term "bioactive compound" refers generally to any physiologically or pharmacologically active substance. In some embodiments, a bioactive agent is a potential therapeutic compound (e.g., small molecule, peptide, nucleic acid, etc.) or drug-like molecule.

As used herein, the term "capture protein" refers to a protein or other molecular entity that forms a stable interaction (e.g., a covalent bond or a stable non-covalent interaction) with its substrate, ligand, or other molecular element upon interaction therewith. A capture protein may be a receptor that forms a covalent bond upon binding its ligand or an enzyme that forms a covalent bond with its substrate. An example of a suitable capture protein for use in embodiments of the present invention is the HALOTAG protein described in U.S. Pat. No. 7,425,436. A capture protein may also be a protein that has a strong non-covalent interaction with its corresponding capture element, such as streptavidin.

As used herein, the term "capture element" refers to a ligand, substrate, etc., that interacts with a corresponding capture protein (e.g., via a covalent bond or a stable non-covalent interaction). An example of a suitable capture element for use in embodiments of the present invention is the HALOTAG ligand described, for example, in U.S. Pat. No. 7,425,436. Moieties that find use as HALOTAG ligands include haloalkane (HA) groups (e.g., chloroalkane (CA) groups). In embodiments described herein that specify an HA or CA capture element, other suitable capture elements may be substituted unless otherwise specified. Another capture element is biotin.

### DETAILED DESCRIPTION

The need to study and map dynamic microenvironments, molecular networks, small molecule interactions as well as protein-protein and protein-nucleic acid interactions in physiologically relevant contexts create a demand for new functional biological tools to enable such analyses in live cells and complex models in a nondestructive fashion. Photoactivatable compounds comprising a functional moiety linked to photoreactive group that can undergo activation upon covalent crosslinking with biomolecules offers a solution to this need by enabling labeling of biomolecules with fluorophores for detection, capture elements for enrichment and identification as well as labeling with a bifunctional moiety comprising a bioactive compound and either a fluorophore or a capture element or a reactive moiety for photoaffinity labeling and subsequent detection and/or enrichment, etc.

Provided herein are compounds, compositions, systems, and methods for photoactivated labeling of biomolecules, which can be actuated within biological systems. In particular, compounds disclosed herein include photoactivatable moieties that generate reactive intermediates upon exposure to light, and subsequently form covalent linkages with biomolecules. The photoactivation can be conducted by a variety of mechanisms including ultraviolet (UV) irradiation, visible light irradiation, or energy transfer. The compounds also include functional moieties that provide useful functionalities, for example detection and/or enrichment of biomolecules, such as fluorophores, capture elements (e.g., biotin), reactive moieties (e.g., click handles), or bifunctional moiety comprising a bioactive compound and either a fluorophore or a capture element or a reactive moiety. These bioorthogonal labeling chemistries can be leveraged for a broad range of phenotypic, proteomic, and genomic analyses.

### Compounds

In accordance with the invention, disclosed herein is a compound of formula (I): 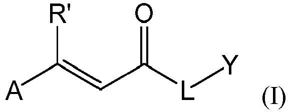 or a salt thereof, wherein:
A is selected from: 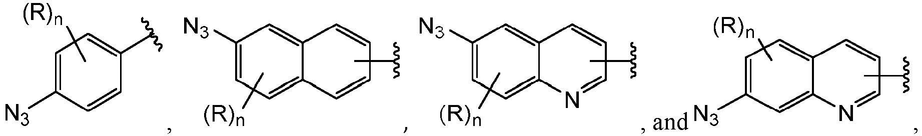 wherein:
   each n is independently 1, 2, 3, or 4; and
   each R is independently selected from hydrogen, halo, C₁-C₄ alkyl, C₂-C₄ alkenyl, hydroxy, mercapto, amino, cyano, C₁-C₄-alkoxy, halo-C₁-C₄-alkyl, hydroxy-C₁-C₄-alkyl, amino-C₁-C₄-alkyl, mercapto-C₁-C₄-alkyl, cyano-C₁-C₄-alkyl, - C(O)-C₁-C₄-alkyl, -C(O)OH, and -C(O)NH₂;
R' is hydrogen or C₁-C₄ alkyl;
L is a linker; and
Y is a functional moiety selected from:
   (a) biotin;
   (b) a haloalkane group;
   (c) a fluorescent functional group selected from a xanthene, a cyanine, a naphthalene, an oxadiazole, a pyrene, an oxazine, an acridine, an arylmethine, a tetrapyrrole, a coumarin, a squaraine, and a boron-dipyrromethene; and
   (d) a reactive functional group comprising an azide, alkyne, alkene, or 1,2,4,5-tetrazinyl moiety.

In some embodiments, A is a group of formula: 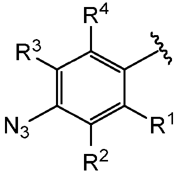 wherein:
R¹, R², R³, and R⁴ are each independently selected from hydrogen, halo, hydroxy, cyano, and C₁-C₄ alkoxy. In some embodiments: R¹ is hydrogen, hydroxy, or C₁-C₄ alkoxy; R² is hydrogen, halo, cyano, or C₁-C₄ alkoxy; R³ is hydrogen or halo; and R⁴ is hydrogen or halo. In some embodiments: R¹ is hydrogen, hydroxy, or methoxy; R² is hydrogen, fluoro, cyano, or methoxy; R³ is hydrogen or fluoro; and R⁴ is hydrogen.

In some embodiments, A is a group of formula: 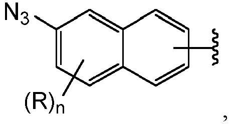 wherein n is 1, 2, or 3, and each R is independently selected from hydrogen, halo, cyano, and C₁-C₄ alkoxy. In some embodiments, n is 1 and R is hydrogen or cyano. In some embodiments, R is hydrogen. In some embodiments, R is cyano.

In some embodiments, In some embodiments, A is a group of formula: 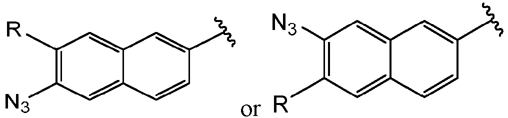 wherein R is selected from hydrogen, halo, cyano, and C₁-C₄ alkoxy. In some embodiments, R is selected from hydrogen and cyano. In some embodiments, R is hydrogen. In some embodiments, R is cyano.

In some embodiments, A is a group of formula: 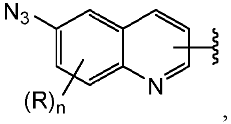 wherein n is 1, 2, or 3, and each R is independently selected from hydrogen, halo, cyano, and C₁-C₄ alkoxy. In some embodiments, n is 1 and R is hydrogen or cyano. In some embodiments, R is hydrogen.

In some embodiments, A is a group of formula: 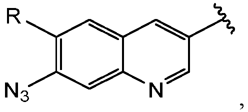 wherein R is hydrogen, halo, cyano, or C₁-C₄ alkoxy. In some embodiments, R is hydrogen or cyano. In some embodiments, R is hydrogen. In some embodiments, R is cyano.

In some embodiments, A is a group of formula: 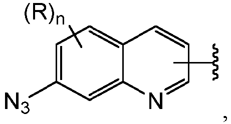 wherein n is 1, 2, or 3, and each R is independently selected from hydrogen, halo, cyano, and C₁-C₄ alkoxy. In some embodiments, n is 1 and R is hydrogen or cyano. In some embodiments, R is hydrogen.

In some embodiments, A is a group of formula: 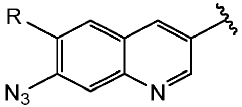 wherein R is hydrogen, halo, cyano, or C₁-C₄ alkoxy. In some embodiments, R is hydrogen or cyano. In some embodiments, R is hydrogen. In some embodiments, R is cyano.

In some embodiments, A has a formula selected from: 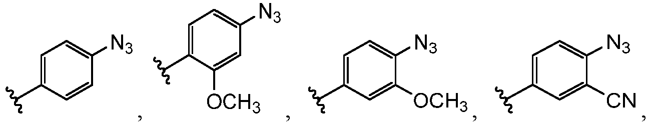 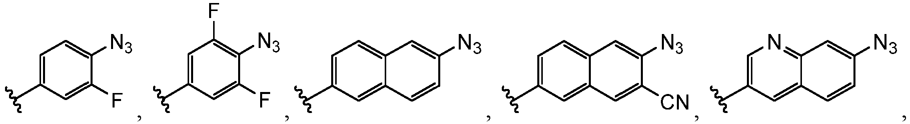 and

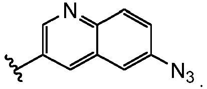

In some embodiments, R' is hydrogen. In some embodiments, R' is C₁-C₄ alkyl. In some embodiments, R' is methyl. In some embodiments, R' is selected from hydrogen and methyl.

The linker can include one or more groups independently selected from methylene (-CH₂-), ethylene (-CH=CH-), ethynylene (-C≡C-), ether (-O-), amine (-NR-), wherein R is hydrogen or an alkyl group), thioether (-S-), carbonyl (-C(O)-), thiocarbonyl (-C(S)-), sulfonyl (-S(O)₂-), arylene, heteroarylene, and heterocyclylene moieties, or any combination thereof. For example, the above moieties can be combined to form additional groups that may be included in the linker, e.g., a carbonyl group and an ether group can together provide an ester moiety (-C(O)O-); a carbonyl group and two ether groups can together provide a carbonate moiety (-OC(O)O-); a carbonyl group and an unsubstituted amine group can together provide an unsubstituted amide moiety (-C(O)NH-); a carbonyl group and two unsubstituted amine groups can together provide an unsubstituted urea moiety (-NHC(O)NH-); a carbonyl group together with an unsubstituted amine group and an ester group can provide an unsubstituted carbamate moiety (-OC(O)NH-); a carbonyl group together with a thioether and an unsubstituted amine group can provide an S-thiocarbamate moiety; a thiocarbonyl group together with an ether and an unsubstituted amine group can provide an O-thiocarbamate moiety; multiple methylene groups can together form an alkylene chain; etc. In some embodiments, the linker comprises one or more methylene, ether, ester, amide, carbamate, carbonate, urea, thioether, thioester, thioamide, thiocarbamate, thiocarbonate, thiourea, arylene, heteroarylene, or heterocyclylene moieties, or any combination thereof. In some embodiments, the linker comprises one or more -CH₂-, -O-, -C(O)O-, -C(O)NH-, -NHC(O)O-, -OC(O)O-, -NHC(O)NH-, -S-, -C(O)S-, -C(S)NH-, -NHC(S)O-, -OC(S)O-, - NHC(S)NH-, arylene, heteroarylene, or heterocyclylene moieties, or any combination thereof.

In some embodiments, the linker comprises one or more moieties selected from straight or branched chain alkylene, -O-, -NH-, -C(O)NH-, -NHC(O)O-, -NHC(O)NH-, and phenylene groups. In some embodiments, the linker comprises one or more moieties selected from straight or branched chain alkylene, -O-, and -NH- groups. In some embodiments, the linker comprises one or more ethylene glycol units (-CH₂CH₂O-).

In some embodiments, the linker has a formula:

-NHCH₂CH₂(OCH₂CH₂)ₙNH-

wherein n is 1, 2, 3, 4, 5, 6, 7, or 8. In some embodiments, n is 3, 4, 5, or 6. In some embodiments, n is 1. In some embodiments, n is 2. In some embodiments, n is 3. In some embodiments, n is 4. In some embodiments, n is 5. In some embodiments, n is 6. In some embodiments, n is 7. In some embodiments, n is 8.

The group Y in compounds of formula (I) is a functional moiety, such as a capture element, a detectable moiety, a reactive moiety, or a bifunctional moiety (e.g., a moiety comprising a bioactive compound and either a reactive moiety, or a capture element, or a fluorophore). In some embodiments, Y is a capture element, which is a group, such as a ligand or a substrate, which forms a covalent or a non-covalent bond with a protein (a "capture protein") upon interaction therewith. In some embodiments, the capture element is a HALOTAG ligand, which is described in, for example, in U.S. Pat. No. 7,425,436. Moieties that find use as HALOTAG ligands include haloalkane (HA) groups (e.g., chloroalkane (CA) groups). For example, in some embodiments, Y has a formula -(CH₂)ₙ-X, wherein n is 2, 3, 4, 5, 6, 7, 8, 9, 10, 11, or 12, and X is a halogen (e.g., chloro). In such embodiments, the corresponding capture protein is the HALOTAG protein, which is described in, for example, in U.S. Pat. No. 7,425,436. Another example of a capture element is biotin. For example, in some embodiments, Y has a formula: 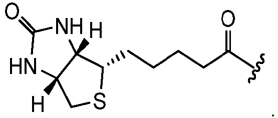 In such embodiments, the corresponding capture protein is, for example, streptavidin.

In some embodiments, Y is a detectable moiety, such as a fluorescent moiety. Suitable fluorescent functional groups include, but are not limited to: xanthene derivatives (e.g., fluorescein, rhodamine, Oregon green, eosin, Texas red, etc.), cyanine derivatives (e.g., cyanine, indocarbocyanine, oxacarbocyanine, thiacarbocyanine, merocyanine, etc.), naphthalene derivatives (e.g., dansyl and prodan derivatives), oxadiazole derivatives (e.g., pyridyloxazole, nitrobenzoxadiazole, benzoxadiazole, etc.), pyrene derivatives (e.g., cascade blue), oxazine derivatives (e.g., Nile red, Nile blue, cresyl violet, oxazine 170, etc.), acridine derivatives (e.g., proflavin, acridine orange, acridine yellow, etc.), arylmethine derivatives (e.g., auramine, crystal violet, malachite green, etc.), tetrapyrrole derivatives (e.g., porphin, phtalocyanine, bilirubin, etc.), CF dye (Biotium), boron dipyrromethenes (BODIPY dyes, Invitrogen), ALEXA FLUOR (Invitrogen), DYLIGHT FLUOR (Thermo Scientific, Pierce), ATTO and TRACY (Sigma Aldrich), FluoProbes (Interchim), DY and MEGASTOKES (Dyomics), SULFO CY dyes (CYANDYE, LLC), SETAU AND SQUARE DYES (SETA BioMedicals), QUASAR and CAL FLUOR dyes (Biosearch Technologies), SURELIGHT DYES (APC, RPE, PerCP, Phycobilisomes)(Columbia Biosciences), APC, APCXL, RPE, BPE (Phyco-Biotech), autofluorescent proteins (e.g., YFP, RFP, mCherry, mKate), quantum dot nanocrystals, etc.

In some embodiments, Y comprises a fluorogenic functional group, which produces enhanced fluorescent signal upon being associated with a target (e.g., binding of a protein to a moiety linked to the fluorogenic functional group). By producing significantly increased fluorescence (e.g., 10X, 20X, 50X, 100X, 200X, 500X, 100X, or more) upon target engagement, background signal is alleviated. Exemplary fluorogenic dyes for use in embodiments herein include the JANELIA FLUOR family of fluorophores, such as:
JANELIA FLUOR 549: 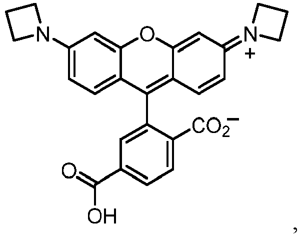
JANELIA FLUOR 646: 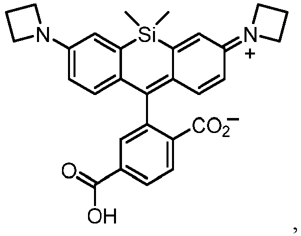
JANELIA FLUOR 585, : 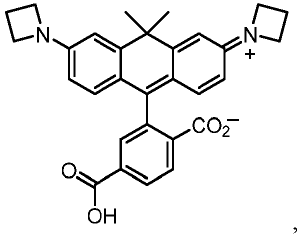
JANELIA FLUOR 635: 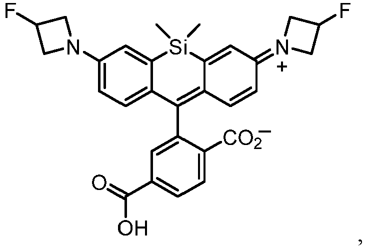 and
JANELIA FLUOR 669:

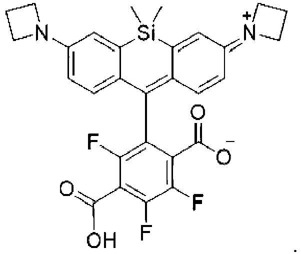

In some embodiments, Y comprises a reactive functional group, which can undergo further reaction with a corresponding reactive moiety on another molecule to effect covalent attachment. For example, in some embodiments, Y comprises a group selected from an azide, an alkyne, an alkene, or a 1,2,4,5-tetrazinyl moiety, all commonly known as "click handles" that can undergo copper-catalyzed or copper-free "click" reactions (e.g., reaction of an azide and an alkyne, reaction of an azide with a difluorinated cyclooctyne, or reaction of a 1,2,4,5-tetrazinyl group with a trans-cyclooctene moiety).

In some embodiments, Y comprises bifunctional moiety comprising a bioactive compound and either a fluorophore or a capture element or a reactive moiety for photoaffinity labeling and subsequent detection and/or enrichment. In some embodiments, the bioactive compound is a small molecule, e.g., a therapeutic agent.

In some embodiments, the compound of formula (I) is a compound selected from: 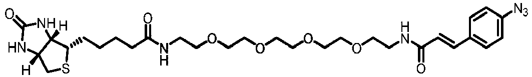 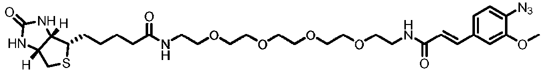 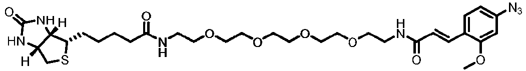 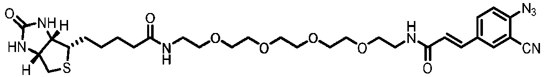 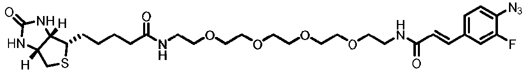 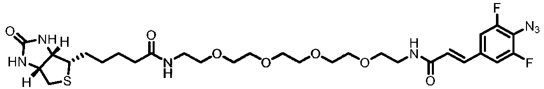 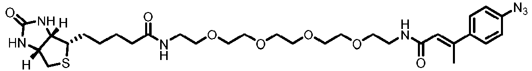 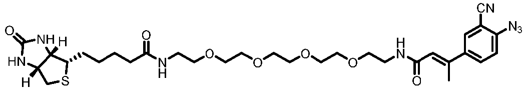 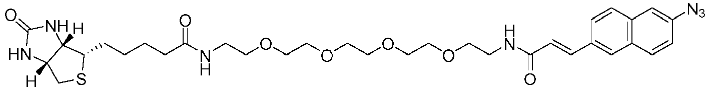 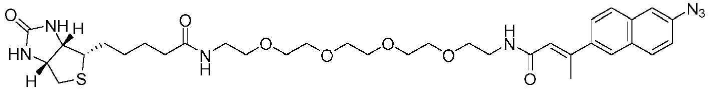 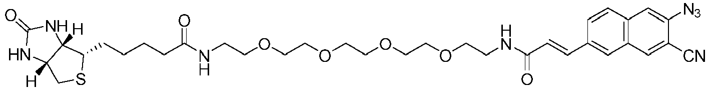 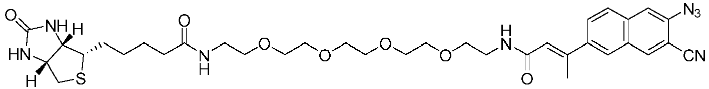 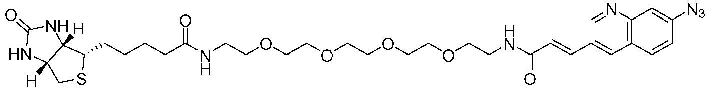 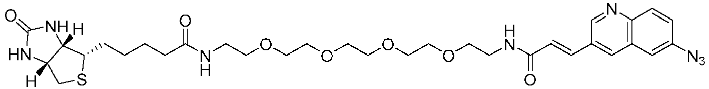 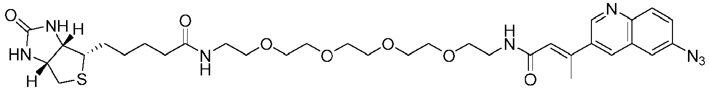 and salts thereof.

The present disclosure also includes isotopically-labeled compounds, which are identical to those recited in formula (I), but for the fact that one or more atoms are replaced by an atom having an atomic mass or mass number different from the atomic mass or mass number usually found in nature. Examples of isotopes suitable for inclusion in the compounds of the invention are hydrogen, carbon, nitrogen, oxygen, phosphorus, sulfur, fluorine, and chlorine, such as, but not limited to, ²H, ³H, ¹³C, ¹⁴C, ¹⁵N, ¹⁸O, ¹⁷O, ³¹P, ³²P, ³⁵S, ¹⁸F, and ³⁶Cl, respectively. Substitution with heavier isotopes such as deuterium, i.e., ²H, can afford certain advantages resulting from greater metabolic stability, for example increased *in vivo* half-life, and may therefore be preferred in some circumstances. Isotopically-labeled compounds of formula (I) can generally be prepared by conventional techniques known to those skilled in the art or by processes analogous to those described in the accompanying examples using appropriate isotopically-labeled reagent in place of non-isotopically-labeled reagent.

A compound disclosed herein may be in the form of a salt. The salts may be prepared during the final isolation and purification of the compounds or separately, for example by reacting a basic group of the compound (e.g., an amino group) with a suitable acid or by reacting an acidic group of the compound (e.g., a carboxylic acid group) with a suitable base.

Acid salts may be prepared during the final isolation and purification of the compounds or separately by reacting a suitable group of the compound, such as an amino group, with a suitable acid. For example, a compound may be dissolved in a suitable solvent, such as, but not limited to, methanol and water, and treated with at least one equivalent of an acid, such hydrochloric acid. The resulting salt may precipitate out and be isolated by filtration and dried under reduced pressure. Alternatively, the solvent and excess acid may be removed under reduced pressure to provide a salt. Representative salts include acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, formate, isethionate, fumarate, lactate, maleate, methane sulfonate, naphthylenesulfonate, nicotinate, oxalate, pamoate, pectinate, persulfate, 3-phenylpropionate, picrate, oxalate, maleate, pivalate, propionate, succinate, tartrate, trichloroacetate, trifluoroacetate, glutamate, para-toluenesulfonate, undecanoate, hydrochloric, hydrobromic, sulfuric, phosphoric and the like. The amino groups of the compounds may also be quaternized with alkyl chlorides, bromides, and iodides such as methyl, ethyl, propyl, isopropyl, butyl, lauryl, myristyl, stearyl and the like.

Basic addition salts may be prepared during the final isolation and purification of the disclosed compounds by reaction of a carboxyl group with a suitable base such as the hydroxide, carbonate, or bicarbonate of a metal cation such as lithium, sodium, potassium, calcium, magnesium, or aluminum, or an organic primary, secondary, or tertiary amine. Quaternary amine salts can be prepared, such as those derived from methylamine, dimethylamine, trimethylamine, triethylamine, diethylamine, ethylamine, tributylamine, pyridine, N,N-dimethylaniline, N-methyl piperidine, N-methylmorpholine, dicyclohexylamine, procaine, dibenzylamine, N,N-dibenzylphenethylamine, 1-ephenamine and N,N'-dibenzylethylenediamine, ethylenediamine, ethanolamine, diethanolamine, piperidine, piperazine, and the like.

The compounds disclosed herein can be synthesized by a variety of methods, including those shown in the Examples. Routine experimentations, including appropriate manipulation of the reaction conditions, reagents and sequence of the synthetic route, protection of any chemical functionality that cannot be compatible with the reaction conditions, and deprotection at a suitable point in the reaction sequence of the method are included in the scope of the disclosure. Suitable protecting groups and the methods for protecting and deprotecting different substituents using such suitable protecting groups are well known to those skilled in the art; examples of which can be found in PGM Wuts and TW Greene, in Greene's book titled Protective Groups in Organic Synthesis (4th ed.), John Wiley & Sons, NY (2006). Synthesis of the compounds of the disclosure can be accomplished by methods analogous to those described in the synthetic schemes described herein and in specific examples.

When an optically active form of a disclosed compound is required, it can be obtained by carrying out one of the procedures described herein using an optically active starting material (prepared, for example, by asymmetric induction of a suitable reaction step) or by resolution of a mixture of the stereoisomers of the compound or intermediates using a standard procedure (such as chromatographic separation, recrystallization, or enzymatic resolution).

Similarly, when a pure geometric isomer of a compound is required, it can be obtained by carrying out one of the above procedures using a pure geometric isomer as a starting material or by resolution of a mixture of the geometric isomers of the compound or intermediates using a standard procedure such as chromatographic separation.

### Photocatalysts

In some embodiments, compounds disclosed herein are used (e.g., in the systems and methods described herein) in conjunction with a photocatalyst that is capable of absorbing light and subsequently activating the photoactivatable aryl-azide moiety on the compound of formula (I). Any compound or moiety capable of receiving light energy and transferring that energy to activate the photoactivatable moiety may find use in embodiments herein. In some embodiments, the excited photocatalyst transfers energy to the compound of formula (I) via Förster Resonance Energy Transfer, Dexter Energy Transfer, Single Electron Transfer, singlet oxygen, or any other suitable mechanism of energy or electron transfer.

In some embodiments, the photocatalyst is an iridium-based or ruthenium-based photocatalyst (Bevernaegie et al. "A Roadmap Towards Visible Light Mediated Electron Transfer Chemistry with Iridium(III) Complexes." ChemPhotoChem 2021, 5, 217. In some embodiments, the photocatalyst is a compound of the following formula: 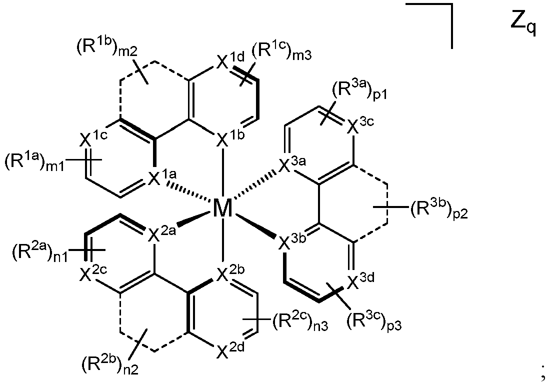 wherein:
each set of dashed lines (------) represents the presence or absence of a fused 6-membered ring;
M is a transition metal;
m1, m2, m3, n1, n2, n3, p1, p2, and p3 are each independently 0, 1, or 2;
R^{1a}, R^{1b}, R^{1c}, R^{2a}, R^{2b}, R^{2c}, R^{3a}, R^{3b}, and R^{3c} are each independently selected from halo, alkyl, haloalkyl, amino, and heteroalkyl;
X^{1a}, X^{1b}, x^{2a}, X^{2b}, X^{3aZ}, and X^{3b} are each independently selected from N and C, wherein at least one of X^{1a} and X^{1b} is N, at least one of X^{2a} and X^{2b} is N, and at least one of X^{3a} and X^{3b} is N;
X^{1c}, X^{1d}, X^{2c}, X^{2d}, X^{3c}, and X^{3d} are each independently selected from CH and N;
Z is an anion; and
q is 0, 1, or 2.

In some embodiments, the photocatalyst comprises a transition metal selected from Ru and Ir.

In some embodiments, the photocatalyst is an iridium-based photocatalyst selected from:

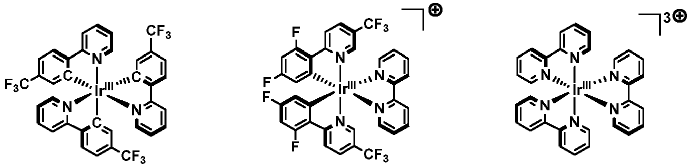

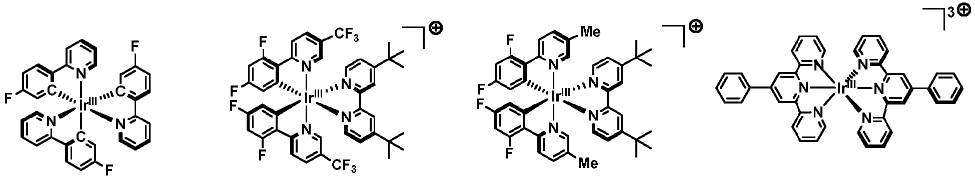

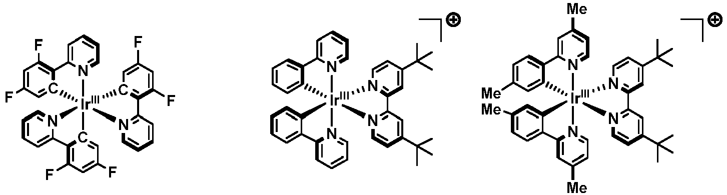

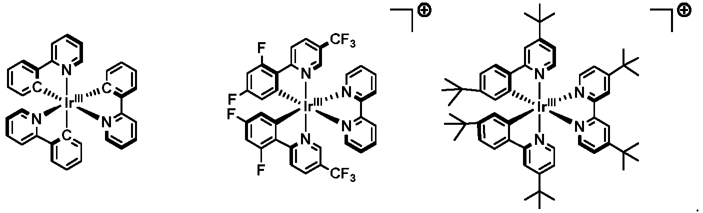

In some embodiments, the photocatalyst is a ruthenium-based photocatalyst selected from:

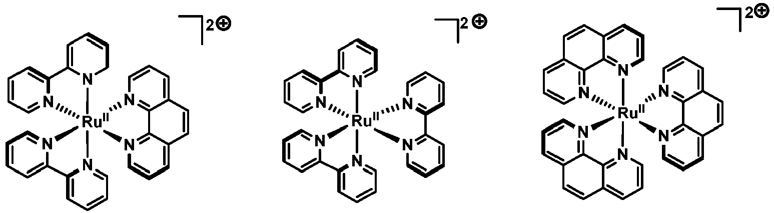

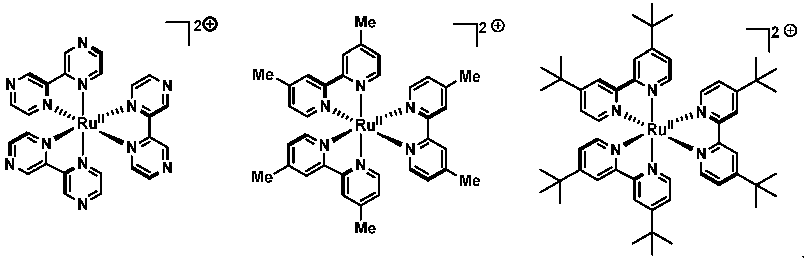

In some embodiments, m2, n2, and p2 are each 0 and each set of dashed lines represents the absence of a fused 6-membered ring, i.e., the compound has formula:

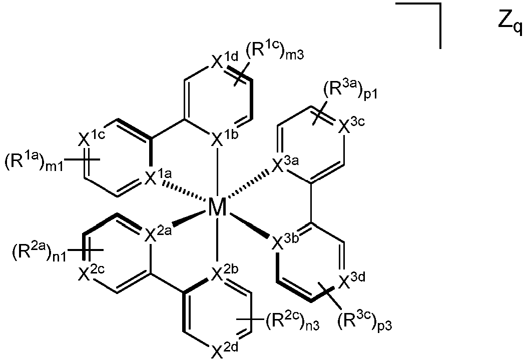

In some embodiments, X^{1a} is N, X^{1b} is C, X^{2a} is N, X^{2b} is C, X^{3a} is C, and X^{3b} is N. In some embodiments, X^{1a} is N, X^{1b} is C, X^{2a} is N, X^{2b} is C, X^{3a} is N, and X^{3b} is N.

In some embodiments, X^{1c}, X^{1d}, X^{2c}, X^{2d}, X^{3c}, and X^{3d} are each CH. In some embodiments, X^{1c}, X^{1d}, X^{2c}, X^{2d}, X^{3c}, and X^{3d} are each N.

In some embodiments, R^{1a}, R^{1b}, R^{1c}, R^{2a}, R^{2b}, R^{2c}, R^{3a}, R^{3b}, and R^{3c} are each independently selected from fluoro, methyl, tert-butyl, and trifluoromethyl. In some embodiments, M is Ru. In some embodiments, M is Ir.

### Systems and Methods of Use

In some embodiments, the present disclosure provides systems and methods for labeling biomolecules using the compounds of formula (I). As discussed above, the compounds of formula (I) include an azide moiety substituted on a phenyl, naphthyl, or quinolinyl group. The aryl azides can undergo light-induced activation to form a reactive nitrene group, which can react with and covalently modify a biomolecule.

Accordingly, the disclosure provides systems and methods for labeling biomolecules, comprising compounds of formula (I). For example, in some embodiments, provided herein is a system for labeling biomolecules, comprising a compound of formula (I) and a photocatalyst. Upon exposure to light, the activated photocatalyst can facilitate energy transfer to the photoactivatable moiety on the compounds of formula (I), promoting generation of the reactive intermediate that covalently labels the biomolecule. Exemplary photocatalysts that can be used in the systems disclosed herein are those disclosed above (e.g., a ruthenium or iridium catalyst described above).

Also, in accordance with the invention, disclosed herein are methods of labeling biomolecules in samples, comprising contacting the sample with a compound of formula (I) and exposing the sample to light. In some embodiments, the light is ultraviolet (UV) light. In some embodiments, the light is visible light. In some embodiments, the light is supplied by a light-emitting diode. In some embodiments, the method further comprises contacting the sample with a photocatalyst, such as a photocatalyst disclosed herein, prior to exposing the sample to light.

In some embodiments, the compounds and systems disclosed herein are used to carry out various methods (e.g., within cells). A variety of functional proteomic and genomic analyses, in addition to other applications, are made possible by the compounds, methods, and systems disclosed herein. Exemplary proteomic focused applications for the systems and methods herein include: proximity-based protein labeling and subsequent detection of dynamic microenvironments; protein-protein interactions and cell-cell interactions under relevant physiological conditions; photoaffinity labeling using a bioactive compound modified with a capture element and the photoreactive group for subsequent enrichment and identification of its cellular targets, ligand directed protein labeling with a fluorophore or click-handle for subsequent attachment of diverse functional groups such as a small molecule drug, PROTAC, etc., for down-stream manipulation of a protein of interest; small molecule drug profiling; etc.

Additional systems and methods using the compounds disclosed herein include systems and methods for bioluminescence-triggered catalysis, such as those disclosed in U.S. Provisional Patent Application No. 63/338,322, filed on May 4, 2022, entitled "BIOLUMINESCENCE-TRIGGERED PHOTOCATALYTIC LABELING,".

### EXAMPLES

### Example 1

Experiments conducted during development of embodiments herein evaluated the capacity of vinyl-aryl-azides, vinyl-naphthyl-azides, and vinyl-quinoline-azides to undergo activation and subsequent covalent crosslink with proteins by UV irradiation, visible 455 nm irradiation as well LED-triggered activation of an iridium catalyst, which further engages in energy transfer events with azide-based photoreactive group (FIGS. 2-5). Structures and absorbance profiles of the vinyl-aryl-azides, vinyl-naphthyl-azides, and vinyl-quinoline-azides are included in FIGS. 2 and FIG. 3, respectively, and their syntheses are described in Example 3.

Absorbance profiles for 200 µM biotin probes comprising vinyl-extended aryl-azide-based photoreactive group in 2% DMSO were monitored on a SPARK multimode plate reader (FIG. 3). Generally, all the vinyl-extended aryl-azide-based photoreactive groups exhibited a red-shifted absorbance relative to a simple phenyl-azide (λmax= ~260 nm) suggesting easier activation by visible blue light and by photocatalytic energy transfer.

The capacity of these vinyl-extended aryl-azide-based photoreactive groups to undergo activation by either UV or visible 455 nm light in the presence or absence of a catalyst for subsequent covalent crosslinking with neighboring proteins was further evaluated through labeling efficiency of a model protein (FIGS. 4 and 5). To this end, reactions comprising either (1) 0.8µM acetylated BSA (Promega) and 50 µM vinyl-extended-aryl-azide-biotin probe or (2) 0.8µM acetylated BSA (Promega), 50 µM vinyl-extended-aryl-azide-biotin probe, and 100 µM iridium catalyst (Ir-9049) were assembled in TE buffer pH 7.5 within wells of a UV transparent 96-well plates. Plates remained in the dark (control) or irradiated using Efficiency Aggregators biophotoreactor for 20 minutes at either 365 nm (100% UV=^{~} 80W) or 455 nm (100% =^{~}80W or 2% =^{~} 2W). To evaluate subsequent covalent labeling efficiencies, reactions were subjected to Zeba column clean-up (ThermoFisher) to remove non-crosslinked vinyl-extended-aryl-azide-biotin probes and then spotted on nitrocellulose membranes using a slot blot apparatus. Membranes were then blocked with 5% BSA (Promega) in TBST for 1 hour at room temperature and subsequently incubated overnight at 4°C with anti-biotin antibody (Invitrogen) in TBST. The next day membranes were washed 3 times with TBST and then incubated for an hour with a secondary anti-goat-HRP-antibody (Jackson laboratories). Following three washes in TBST, membranes were treated with ECL substrate (Promega) and scanned on the chemiluminescence channel to detect BSA labeled with biotin. Western analyses and quantitation of bands volumes using Image J software revealed that all the vinyl-extended-aryl-azide photoreactive groups were activated directly by either UV or 455nm, albeit with different efficiencies. Out the fifteen tested vinyl-extended-aryl-azide photoreactive groups, eight exhibited greater UV activation efficiencies, five exhibited equal UV and 455 nm activation efficiencies, and two, 9616 and 9582, exhibited greater 455 nm activation efficiencies. Interestingly, both are vinyl-naphthyl-azides with a methyl substitution on the vinyl group. Furthermore, iridium catalyst activated by either 100% or 2% LED was capable of engaging in photocatalytic energy transfer events with all fifteen tested vinyl-extended-aryl-azide photoreactive groups resulting in greater labeling efficiencies compared to direct LED activation (i.e., in the absence of a catalyst). Quantitated band volumes were further normalized to the 9157 UV-induced labeling (common in all membranes) revealing a broad range of activation and labeling efficiencies.

### Example 2

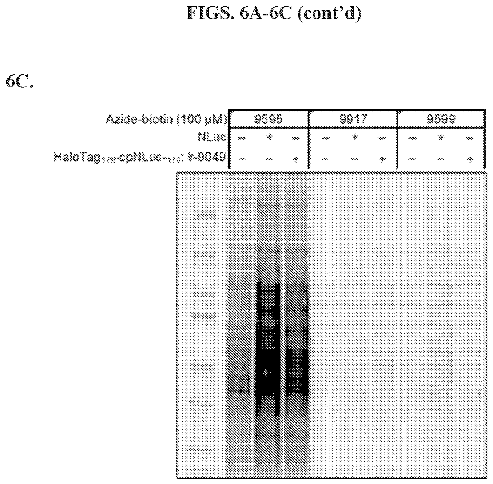

Experiments conducted during development of embodiments herein evaluated the capacity of vinyl-aryl-azides, vinyl-naphthyl-azides, and vinyl-quinoline-azides to undergo bioluminescence-triggered photocatalytic activation and subsequent covalent crosslink with nearby proteins (FIG. 6). Structures and absorbance profiles of the vinyl-aryl-azides, vinyl-naphthyl-azides, and vinyl-quinoline-azides are included in FIGS. 2 and 3, and their syntheses are described in Example 3.

The capacity of bioluminescence-triggered photocatalytic complex to undergo energy transfer events with the vinyl-extended-aryl azide-biotin probes for subsequent crosslinking with proximal proteins was further investigated. The photocatalytic complex (i.e., HaloTag₁₇₈-cpNLuc-₁₇₉:Ir-9049) consist of a HaloTag₁₇₈-cpNLuc-₁₇₉ chimera comprising a circularly permuted NanoLuc (i.e., cpNLuc) inserted into a HaloTag's surface loop (between residues 178-179), which is proximal to the ligand binding site, and a chloroalkane-iridium catalyst conjugate (Ir-9049) that is bound to the chimera and can be activated via biolumincence energy transfer. Briefly, reactions comprising 100µM vinyl-extended-aryl azide-biotin probes, 0.1mg/mL K562 cell lysate depleted of biotinylated proteins, and 60 nM HT₁₇₈-cpNLuc-₁₇₉: Ir-9049 conjugate were assembled in TBS pH 7.5 within wells of a white 96-well plate. Bioluminescence was induced upon treatment with 100µM fluorofurimazine for 20 minutes while control wells remained untreated (light-independent background). In some cases, an additional light-dependent background control was included in which the HT₁₇₈-cpNLuc-₁₇₉: Ir-9049 conjugate was exchanged with NanoLuc (no catalyst). To evaluate labeling efficiencies, samples were collected, resolved on SDS-PAGE, and transferred to nitrocellulose membranes. The membranes were blocked with 5% BSA (Promega) in TBST for 1 hour at room temperature and subsequently incubated overnight at 4°C with Streptavidin-HRP (Invitrogen) in TBST supplemented with 5% BSA. Following three washes in TBST, membranes were treated with ECL substrate (Promega) and scanned on the chemiluminescence channel to detect proteins labeled with biotin. Western analyses demonstrated that these vinyl-extended-aryl-azide-photoreactive groups can undergo activation by bioluminescence-triggered photocatalytic energy transfer for subsequent crosslinking to proximal proteins albeit at a broad range of efficiencies and light-dependent and independent-backgrounds. Notably, the extent of red-shift absorbance for these vinyl-azide-photoreactive groups were associated with increased capacity to undergo activation by bioluminescence-triggered photocatalytic energy transfer. In addition, several substitutions increased labeling specificity to different extents likely by impeding the generation-kinetics and /or lifetime of the reactive intermediates resulting with an overall smaller labeling radius.

### Example 3: Compound Syntheses

### Synthesis of Aryl Azide Intermediates

### Synthesis of A-1 (E)-3-(6-azidonaphthalen-2-yl)acrylic acid

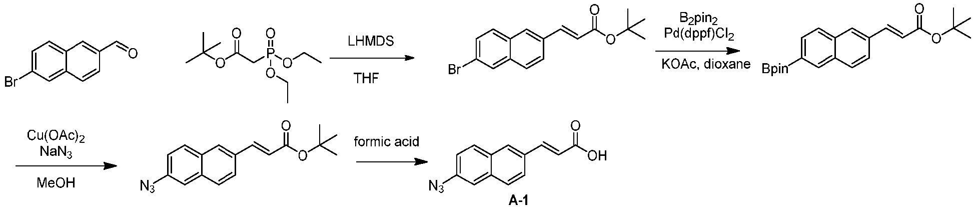

*Step 1:* To a 20 mL vial, the *tert-butyl* 2-(diethoxyphosphoryl)acetate (0.475 mL, 2.13 mmol) and THF (6 mL) was added. The mixture was stirred under nitrogen, and 1M LHMDS in THF (2.13 mL, 2.13 mmol) added dropwise over 5 min. To the mixture, 6-bromo-2-naphthaldehyde (500 mg, 2.13 mmol) was added over 1 min. After 20 min, the mixture was adsorbed to Celite and purified by silica gel chromatography with 0-30% EtOAc in heptane as eluent to afford *tert-butyl* (*E*)-3-(6-bromonaphthalen-2-yl)acrylate. LRMS [M+H-C₄H₄]⁺ 277.

*Step 2:* To a 20 mL vial, tert-butyl (E)-3-(6-bromonaphthalen-2-yl)acrylate (200 mg, 0.600 mmol), Pd(dppf)Cl₂ (22.0 mg, 0.030 mmol), B₂pin₂ (183 mg, 0.720 mmol), potassium acetate (118 mg, 1.20 mmol), and dioxane (4 mL) were added. The mixture was degassed with nitrogen for 1 min. The mixture was stirred and heated at 100°C for 1 h. The mixture was cooled to RT. The mixture was diluted in EtOAc and filtered through Celite. The solvents were evaporated to afford *tert-butyl* (£)-3-(6-(4,4,5,5-tetramethyl-1,3,2-dioxaborolan-2-yl)naphthalen-2-yl)acrylate, which was taken forward to the next step without further purification. LRMS [M+H-C₄H₄]⁺ 325.

*Step 3:* To a 20 mL vial, *tert-butyl* (E)-3-(6-(4,4,5,5-tetramethyl-1,3,2-dioxaborolan-2-yl)naphthalen-2-yl)acrylate (225 mg, 0.592 mmol), NaN₃ (57.8 mg, 0.889 mmol), Cu(OAc)₂ (215 mg, 1.18 mmol), and MeOH (3 mL) was added. The mixture was vigorously at 65°C for 90 min. The mixture was diluted with EtOAc and washed with 10% ammonia. The organic layer was dried over sodium sulfate, filtered, and the solvents were evaporated. The residue was purified by silica gel chromatography with 0-30% EtOAc in heptane as eluent to afford *tert-butyl (E)-3-(6-*azidonaphthalen-2-yl)acrylate. LRMS [M+H-C₄H₄]⁺ 240.

*Step 4:* To a 20 mL vial, *tert*-butyl (*E*)-3-(6-azidonaphthalen-2-yl)acrylate (100 mg, 0.339 mmol), DCM (2 mL), and formic acid (1 mL) were added. The mixture was stirred for 14h. Solids precipitated. The solids were collected by filtration and washed with DCM to afford A-1 (E)-3-(6-azidonaphthalen-2-yl)acrylic acid. LRMS [M-H]⁻ 238.

### Synthesis of A-2 (E)-3-(6-azidonaphthalen-2-yl)but-2-enoic acid

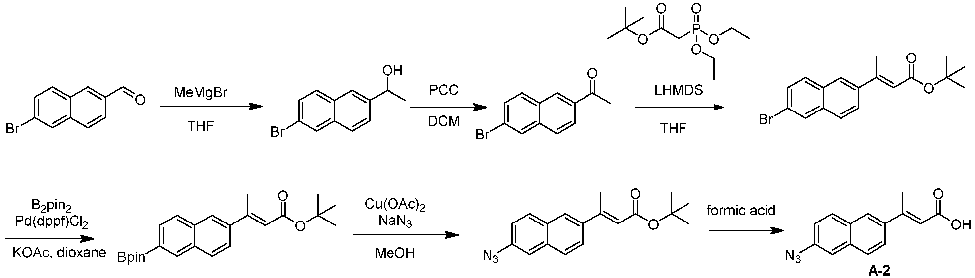

*Step 1:* To a 20 mL vial, 6-bromo-2-naphthaldehyde (500 mg, 2.13 mmol) and THF (10 mL) was added. The mixture was stirred under nitrogen at 0°C. To the mixture, a 1.4 M solution of MeMgBr (1.82 mL, 2.55 mmol) was added over 5 min. The mixture was stirred for 10 min. The mixture was quenched with saturated ammonium chloride (~0.5 mL) and filtered through Celite. The organic layer was diluted in diethyl ether and washed with water. The organic layer was dried over magnesium sulfate, filtered, and the solvents were evaporated. The residue was purified by silica gel chromatography with 0-30% EtOAc in heptane as eluent to afford 1-(6-bromonaphthalen-2-yl)ethan-1-ol. LRMS [M+H-H₂O]⁺ 233.

*Step 2:* To a 100 mL flask, PCC (1.09 g, 5.07 mmol), Celite (2.5 g), and DCM (20 mL) was added. To the stirring mixture, 1-(6-bromonaphthalen-2-yl)ethan-1-ol (424 mg, 1.69 mmol) was added. The mixture was stirred at room temperature for 1h. The mixture was filtered through Celite, washing with DCM. The solvents of the filtrate were evaporated. The residue was purified by silica gel chromatography with 0-30% EtOAc in heptane as eluent to afford 1-(6-bromonaphthalen-2-yl)ethan-1-one. LRMS [M+H]⁺ 249.

*Step 3:* To a 20 mL vial, the *tert-butyl* 2-(diethoxyphosphoryl)acetate (0.307 mL, 1.38 mmol) and THF (5 mL) was added. The mixture was stirred under nitrogen. To the mixture, 1M LHMDS in THF (1.38 mL, 1.38 mmol) was added dropwise over 5 min. To the mixture, a solution of 1-(6-bromonaphthalen-2-yl)ethan-1-one (343 mg, 1.38 mmol) in THF was added dropwise over 5 min. After 5 min, the vial was sealed then stirred and heated at 70°C for 3h. The mixture was adsorbed to Celite and purified by silica gel chromatography with 0-20% EtOAc in heptane as eluent to afford *tert*-butyl (*E*)-3-(6-bromonaphthalen-2-yl)but-2-enoate. LRMS [M+H-C₄H₄]⁺ 291.

*Step 4:* To a 20 mL vial, tert-butyl (E)-3-(6-bromonaphthalen-2-yl)but-2-enoate (350 mg, 1.01 mmol), Pd(dppf)Cl₂ (73.8 mg, 0.101 mmol), B₂pin₂ (307 mg, 1.21 mmol), potassium acetate (198 mg, 2.02 mmol), and dioxane (5 mL) were added. The mixture was degassed with nitrogen for 1 min. The mixture was stirred and heated at 120°C for 2.5h. The mixture was cooled to RT. The mixture was diluted in EtOAc and filtered through Celite. The solvents were evaporated to afford *tert-butyl* (*E*)-3-(6-(4,4,5,5-tetramethyl-1,3,2-dioxaborolan-2-yl)naphthalen-2-yl)but-2-enoate, which was taken forward to the next step without further purification. LRMS [M+H-C₄H₄]⁺ 339.

*Step 5:* To a 20 mL vial, *tert-butyl* (*E*)-3-(6-(4,4,5,5-tetramethyl-1,3,2-dioxaborolan-2-yl)naphthalen-2-yl)but-2-enoate (145 mg, 0.368 mmol), NaN₃ (35.9 mg, 0.552 mmol), Cu(OAc)₂ (134 mg, 0.736 mmol), and MeOH (8 mL) was added. The mixture was vigorously at 65°C for 90 min. The mixture was diluted with EtOAc and washed with 10% ammonia. The organic layer was dried over sodium sulfate, filtered, and the solvents were evaporated. The residue was purified by silica gel chromatography with 0-50% EtOAc in heptane as eluent to afford tert-butyl (E)-3-(6-azidonaphthalen-2-yl)but-2-enoate. LRMS [M+H-C₄H₄]⁺ 254.

*Step 6:* To a 20 mL vial, tert-butyl (*E*)-3-(6-azidonaphthalen-2-yl)but-2-enoate (30.0 mg, 0.0970 mmol), DCM (2 mL), and formic acid (1 mL) was added. The mixture was stirred for 14h. The solvents were evaporated to afford **A-2** (*E*)-3-(6-azidonaphthalen-2-yl)but-2-enoic acid. LRMS [M-H]⁻ 252.

### Synthesis of A-3 (E)-3-(7-azidoquinolin-3-yl)acrylic acid

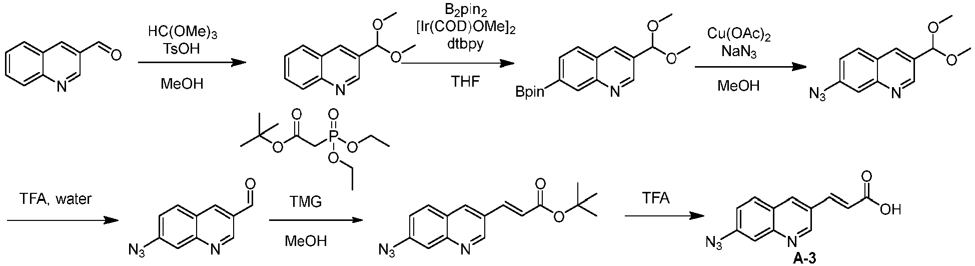

*Step 1:* To a 100 mL round bottom flask, quinoline-3-carbaldehyde (2.00 g, 12.7 mmol), p-toluenesulfonic acid monohydrate (219 mg, 1.27 mmol), MeOH (15 mL), and the trtimethylorthoformate (13.9 mL, 127 mmol) was added. The mixture was stirred and heated at 70°C for 3 hours. The mixture was concentrated and purified by silica gel chromatography with 0-70% EtOAc in heptane as eluent to afford 3-(dimethoxymethyl)quinoline. LRMS [M+H]⁺ 204.

*Step 2:* To a 20 mL vial, 3-(dimethoxymethyl)quinoline (500 mg, 2.46 mmol), [Ir(COD)OMe]₂ (81.5 mg, 0.123 mmol), B₂pin₂ (937 mg, 3.69 mmol), and *4,4'-di-tert-*butylbipyridine (dtbpy, 66.0 mg, 0.246 mmol) was added. The vial was purged with nitrogen. To the mixture, dry THF (5 mL) was added. The mixture was sparged with nitrogen for 1 min. The mixture was stirred at RT for 14h. The solvents was evaporated to afford crude 3-(dimethoxymethyl)-7-(4,4,5,5-tetramethyl-1,3,2-dioxaborolan-2-yl)quinoline, which was taken to the next step without purification. LRMS [M+H]⁺ 330.

*Step 3:* To a 20 mL vial, 3-(dimethoxymethyl)-7-(4,4,5,5-tetramethyl-1,3,2-dioxaborolan-2-yl)quinoline (810 mg, 2.46 mmol), NaN₃ (240 mg, 3.69 mmol), Cu(OAc)₂ (894 mg, 4.62 mmol), and MeOH (8 mL) was added. The mixture was stirred vigorously at 55°C for 90 min. The mixture was diluted with EtOAc and washed with 10% ammonia. The organic layer was dried over sodium sulfate, filtered, and the solvents were evaporated. The residue was purified by silica gel chromatography with 0-40% EtOAc in heptane as eluent to afford 7-azido-3-(dimethoxymethyl)quinoline. LRMS [M+H]⁺ 245.

*Step 4:* To a 20 mL vial, 7-azido-3-(dimethoxymethyl)quinoline (26.9 mg, 0.110 mmol), TFA (1 mL), and water (0.1 mL) was added. The mixture was stirred for 10 min. The solvents were evaporated to afford 7-azidoquinoline-3-carbaldehyde. LRMS [M+H]⁺ 199.

*Step 5:* To a 20 mL vial, *tert*-butyl 2-(diethoxyphosphoryl)acetate (0.0278 mL, 0.110 mmol), 7-azidoquinoline-3-carbaldehyde (21.8 mg, 0.110 mmol), and MeOH (1 mL) was added. To the stirring mixture, tetramethylguanidine (TMG, 0.055 mL, 0.441 mmol) was added dropwise. After 20 min, the solvents were evaporated, and the residue was purified by silica gel chromatography with 0-50% EtOAc in heptane as eluent to afford tert-butyl (E)-3-(7-azidoquinolin-3-yl)acrylate. LRMS [M+H]⁺ 297.

*Step 6:* To a 20 mL vial, tert-butyl (*E*)-3-(7-azidoquinolin-3-yl)acrylate (23.7 mg, 0.0800 mmol) and TFA (1 mL) was added. The mixture was stirred for 15 min. The solvents were evaporated to afford **A-3** (*E*)-3-(7-azidoquinolin-3-yl)acrylic acid. LRMS [M+H]⁺ 241.

### Synthesis of A-4 (E)-3-(6-azidoquinolin-3-yl)acrylic acid

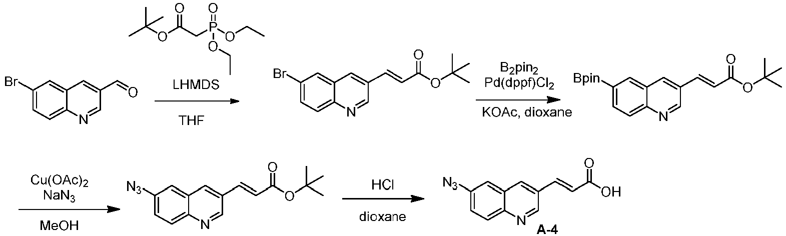

*Step 1:* To a 20 mL vial, the tert-butyl 2-(diethoxyphosphoryl)acetate (0.378mL, 1.69 mmol) and THF (6 mL) was added. The mixture was stirred under nitrogen. To the mixture, 1M LHMDS in THF (1.69 mL, 1.69 mmol) was added dropwise over 5 min. To the mixture, 6-bromoquinoline-3-carbaldehyde (Cheng, Yuan et al. WO2011063233 A1) (400 mg, 1.69 mmol) was added over 1 min. After 20 min, the mixture was adsorbed to Celite and purified by silica gel chromatography with 0-50% EtOAc in heptane as eluent to afford tert-butyl (E)-3-(6-bromoquinolin-3-yl)acrylate. LRMS [M+H]⁺ 334.

*Step 2:* To a 20 mL vial, *tert-butyl* (*E*)-3-(6-bromoquinolin-3-yl)acrylate (64.0 mg, 0.191 mmol), Pd(dppf)Cl₂ (7.0 mg, 0.0096 mmol), B₂pin₂ (58.4 mg, 0.230 mmol), potassium acetate (37.6 mg, 0.383 mmol), and dioxane (4 mL) were added. The mixture was degassed with nitrogen for 1 min. The mixture was stirred and heated at 100°C for 2h. The mixture was cooled to RT. The mixture was diluted in EtOAc and filtered through Celite. The solvents were evaporated to afford *tert*-butyl (£)-3-(6-(4,4,5,5-tetramethyl-1,3,2-dioxaborolan-2-yl)quinolin-3-yl)acrylate, which was taken forward to the next step without further purification. LRMS [M+H]⁺ 382.

*Step 3:* To a 20 mL vial, *tert*-butyl (E)-3-(6-(4,4,5,5-tetramethyl-1,3,2-dioxaborolan-2-yl)quinolin-3-yl)acrylate (73.0 mg, 0.191 mmol), NaN₃ (18.7 mg, 0.287 mmol), Cu(OAc)₂ (69.6 mg, 0.383 mmol), and MeOH (3 mL) was added. The mixture was vigorously at 65°C for 90 min. The mixture was diluted with EtOAc and washed with 10% ammonia. The organic layer was dried over sodium sulfate, filtered, and the solvents were evaporated. The residue was purified by silica gel chromatography with 0-50% EtOAc in heptane as eluent to afford *tert-butyl (E)-3-(6-*azidoquinolin-3-yl)acrylate. LRMS [M+H]⁺ 297.

*Step 4:* To a 20 mL vial, tert-butyl (*E*)-3-(6-azidoquinolin-3-yl)acrylate and 4 M HCl in dioxane (2 mL) was added. The mixture was stirred and heated at 70°C for 2h. The solvents were concentrated to afford **A-4 (***E*)-3-(6-azidoquinolin-3-yl)acrylic acid. LRMS [M+H]⁺ 241.

### Synthesis of A-5 (E)-3-(6-azidoquinolin-3-yl)but-2-enoic acid

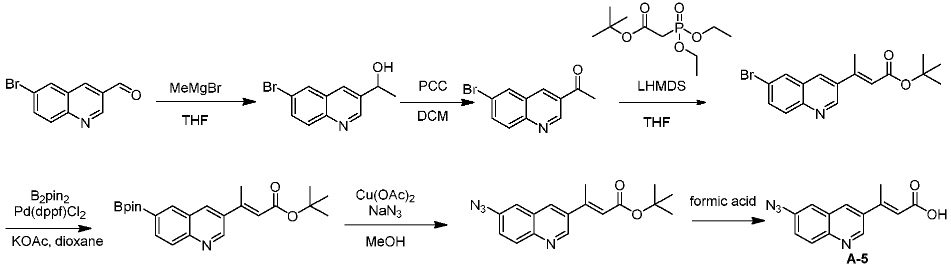

*Step 1:* To a 20 mL vial, 6-bromoquinoline-3-carbaldehyde (Cheng, Yuan et al. WO2011063233 A1) (334 mg, 1.42 mmol) and THF (8 mL) was added. The mixture was stirred under nitrogen at 0°C. To the mixture was added a 1.4 M solution of MeMgBr (1.52 mL, 2.12 mmol) over 5 min. The mixture was stirred for 10 min. The mixture was quenched with saturated ammonium chloride (1 mL). The mixture was diluted with EtOAc and filtered through Celite. The filtrate was concentrated, and the residue was purified by silica gel chromatography with 0-70% EtOAc in heptane as eluent to afford 1-(6-bromoquinolin-3-yl)ethan-1-ol. LRMS [M+H]⁺ 252.

*Step 2:* To a 20 mL vial, 1-(6-bromoquinolin-3-yl)ethan-1-ol (260 mg, 1.03 mmol), PCC (668 g, 3.10 mmol), Celite (1 g), and DCM (6 mL) was added. The mixture was stirred at room temperature for 2h. The mixture was filtered through Celite, washing with DCM. The solvents of the filtrate were evaporated. The residue was purified by silica gel chromatography with 0-70% EtOAc in heptane as eluent to afford 1-(6-bromoquinolin-3-yl)ethan-1-one. LRMS [M+H]⁺ 250.

*Step 3:* To a 20 mL vial, the *tert-butyl* 2-(diethoxyphosphoryl)acetate (0.185 mL, 0.829 mmol) and THF (6 mL) was added. The mixture was stirred under nitrogen. To the mixture, 1 M LHMDS in THF (1.13 mL, 1.13 mmol) was added dropwise over 5 min. To the mixture, a solution of 1-(6-bromoquinolin-3-yl)ethan-1-one (188 mg, 0.753 mmol) in THF was added dropwise over 5 min. The mixture was stirred for 20 min. The mixture was adsorbed to Celite and purified by silica gel chromatography with 0-50% EtOAc in heptane as eluent to afford tert-butyl (E)-3-(6-bromoquinolin-3-yl)but-2-enoate. LRMS [M+H]⁺ 348.

*Step 4:* To a 20 mL vial, *tert-butyl* (*E*)-3-(6-bromoquinolin-3-yl)but-2-enoate (55.0 mg, 0.158 mmol), Pd(dppf)Cl₂ (5.8 mg, 0.0079 mmol), B₂pin₂ (48.1 mg, 0.190 mmol), potassium acetate (31.0 mg, 0.316 mmol), and dioxane (1 mL) were added. The mixture was degassed with nitrogen for 1 min. The mixture was stirred and heated at 100°C for 2h. The mixture was cooled to RT. The mixture was diluted in EtOAc and filtered through Celite. The solvents were evaporated to afford *tert-butyl* (*E*)-3-(6-(4,4,5,5-tetramethyl-1,3,2-dioxaborolan-2-yl)quinolin-3-yl)but-2-enoate, which was taken forward to the next step without further purification. LRMS [M+H]⁺ 396.

*Step 5:* To a 20 mL vial, *tert-butyl* (E)-3-(6-(4,4,5,5-tetramethyl-1,3,2-dioxaborolan-2-yl)quinolin-3-yl)but-2-enoate (62.4 mg, 0.158 mmol), NaN₃ (15.4 mg, 0.237 mmol), Cu(OAc)₂ (57.3 mg, 0.316 mmol), and MeOH (3 mL) was added. The mixture was vigorously at 65°C for 90 min. The mixture was diluted with EtOAc and washed with 10% ammonia. The organic layer was dried over sodium sulfate, filtered, and the solvents were evaporated. The residue was purified by silica gel chromatography with 0-50% EtOAc in heptane as eluent to afford *tert*-butyl (*E*)-3-(6-azidoquinolin-3-yl)but-2-enoate. LRMS [M+H]⁺ 311.

*Step 6:* To a 20 mL vial, *tert*-butyl (*E*)-3-(6-azidoquinolin-3-yl)but-2-enoate (43.5 mg, 0.140 mmol) and 4 M HCl in dioxane (2 mL) was added. The mixture was stirred and heated at 70°C for 2h. The solvents were evaporated to afford **A-5** (*E*)-3-(6-azidoquinolin-3-yl)but-2-enoic acid. LRMS [M+H]⁺ 255.

### Synthesis of A-6 (E)-3-(6-azido-7-cyanonaphthalen-2-yl)acrylic acid

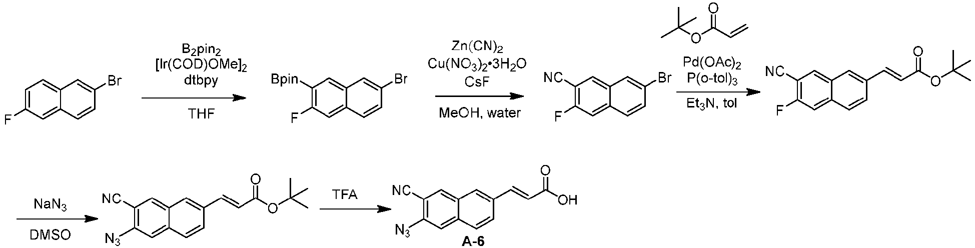

*Step 1:* To a 100 mL flask, 2-bromo-6-fluoronaphthalene (1.00 g, 4.44 mmol), [Ir(COD)OMe]₂ (147 mg, 0.222 mmol), B₂pin₂ (1.69 g, 6.66 mmol), and 4,4'-di-tert-butylbipyridine (dtbpy, 119 mg, 0.444 mmol) was added. The vial was purged with nitrogen. To the mixture, dry THF (10 mL) was added. The mixture was sparged with nitrogen for 1 min. The mixture was stirred at RT for 14 h. The solvents were evaporated. The residue was purified by silica gel chromatography to afford 2-(7-bromo-3-fluoronaphthalen-2-yl)-4,4,5,5-tetramethyl-1,3,2-dioxaborolane. ¹H NMR (400 MHz, DMSO-*d*₆) δ 8.54 (d, *J=* 2.2 Hz, 1H), 8.38 (dd, *J= 4.3,* 2.2 Hz, 2H), 8.33 (d, *J=* 6.0 Hz, 1H), 8.21 (d, *J=* 11.6 Hz, 1H), 8.03 (d, *J=* 2.2 Hz, 1H), 7.89 (d, J= 8.8 Hz, 1H), 7.76 - 7.65 (m, 2H), 1.39 (s, 12H).

*Step 2:* To a 100 mL flask, 2-(7-bromo-3-fluoronaphthalen-2-yl)-4,4,5,5-tetramethyl-1,3,2-dioxaborolane (1.18 g, 3.35 mmol), Cu(NO₃)₂•3H₂O (1.62 g, 6.70 mmol), Zn(CN)₂ (1.18 g, 10.1 mmol), CsF (509 mg, 3.35 mmol), MeOH (20 mL), and water (8 mL) were added. The reaction was stirred and heated at reflux for 1hr. The mixture was cooled to RT. The mixture was diluted in EtOAc and washed with 1M aqueous ammonia. The organic layer was dried over sodium sulfate, filtered, and the solvents of the filtrate were evaporated. The residue was purified by silica gel chromatography with 0-20% EtOAc in heptane to afford 7-bromo-3-fluoro-2-naphthonitrile. ¹H NMR (400 MHz, DMSO-*d*₆) δ 8.71 (d, *J =* 6.6 Hz, 1H), 8.39 (d, *J =* 1.9 Hz, 1H), 8.11 (d, *J =* 10.6 Hz, 1H), 8.02 (d, *J =* 8.9 Hz, 1H), 7.91 (dd, *J =* 8.8, 2.0 Hz, 1H).

*Step 3:* To a 20 mL vial, 7-bromo-3-fluoro-2-naphthonitrile (199 mg, 0.797 mmol), t-buytl acrylate (0.146 mL, 0.996 mmol), triethylamine (0.222 mL, 1.59 mmol), Pd(OAc)₂ (1.8 mg, 0.0080 mmol), tri-(o-tolyl)phosphine (9.7 mg, 0.032 mmol), and toluene (8 mL) was added. The mixture was purged with nitrogen for 1 min. The mixture was stirred and heated at 100°C under nitrogen for 3h. The solvents were evaporated, and the residue was purified by silica gel chromatography with 0-20% EtOAc in heptane to afford *tert*-butyl (*E*)-3-(7-cyano-6-fluoronaphthalen-2-yl)acrylate. LRMS [M+H+MeCN]⁺ 339.

*Step 4:* To a 20 mL vial, the *tert*-butyl (*E*)-3-(7-cyano-6-fluoronaphthalen-2-yl)acrylate (64.7 mg, 0.218 mmol), NaN₃ (15.5 mg, 0.239 mmol), and DMSO (1 mL) was added. The mixture was stirred and heated at 100°C for 2h. The mixture was diluted in 1:2 EtOAc/Et₂O (12 mL) and filtered through Celite, rinsing with Et₂O. The filtrate was washed with water (3 x 10 mL). The organic layer was dried over sodium sulfate, filtered, and the solvents of the filtrate were evaporated. The mixture was purified by silica gel chromatography with 0-30% EtOAc in heptane as eluent to afford the *tert*-butyl (*E*)-3-(6-azido-7-cyanonaphthalen-2-yl)acrylate. LRMS [M+H-N₂]⁺ 293.

*Step 5:* To a 20 mL vial, *tert*-butyl (*E*)-3-(6-azido-7-cyanonaphthalen-2-yl)acrylate (37.4 mg, 0.117 mmol) and TFA (1 mL) was added. The mixture was stirred for 15 min. The solvents were evaporated to afford **A-6** (*E*)-3-(6-azido-7-cyanonaphthalen-2-yl)acrylic acid. LRMS [M-H]⁻263.

### Synthesis of A-7 (E)-3-(6-azido-7-cyanonaphthalen-2-yl)but-2-enoic acid

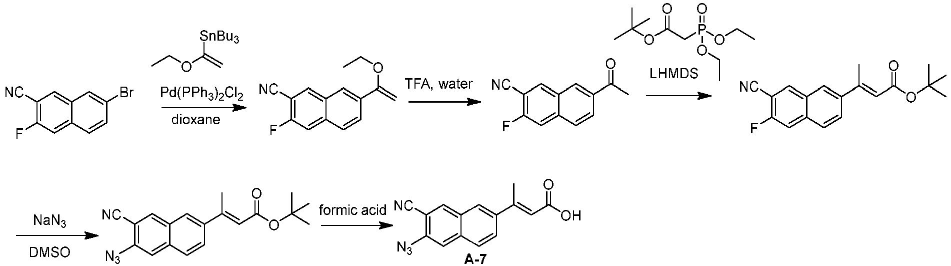

*Step 1:* To a solution of 7-bromo-3-fluoro-2-naphthonitrile (from *Step 2* of **A-6**) (266 mg, 1.07 mmol) in dioxane (2 mL), tributyl(1-ethoxyvinyl)tin (0.396 mL, 1.17 mmol), and Pd(PPh₃)₂Cl₂ (37.4 mg, 0.0533 mmol) was added. The mixture was purged with nitrogen for 2 min. The mixture was stirred and heated at 130°C for 30 min. The mixture was cooled to RT, and the mixture was diluted in EtOAc and filtered through Celite. The solvents of the filtrate were evaporated. The residue was purified by silica gel chromatography with 0-30% EtOAc in heptane as eluent to afford 7-(1-ethoxyvinyl)-3-fluoro-2-naphthonitrile. LRMS [M+H]⁺ 242.

*Step 2:* To a 20 mL vial, 7-(1-ethoxyvinyl)-3-fluoro-2-naphthonitrile (154 mg, 0.637 mmol) and 10% v/v water in TFA (2 mL) was added and stirred for 10 mins. The solvents were evaporated to afford 7-acetyl-3-fluoro-2-naphthonitrile.

*Step 3:* To a 20 mL vial, the *tert*-butyl 2-(diethoxyphosphoryl)acetate (0.152 mL, 0.679 mmol) and THF (3 mL) was added. The mixture was stirred under nitrogen. To the mixture, 1M LHMDS in THF (0.679 mL, 0.679 mmol) was added dropwise over 5 min. To the mixture, a solution of 7-acetyl-3-fluoro-2-naphthonitrile (145 mg, 0.679 mmol) in THF was added dropwise over 5 min. After 5 min, the vial was sealed then stirred and heated at 70°C for 14h. The mixture was adsorbed to Celite and purified by silica gel chromatography with 0-30% EtOAc in heptane as eluent to afford *tert-butyl* (*E*)-3-(7-cyano-6-fluoronaphthalen-2-yl)but-2-enoate. LRMS [M+H+MeCN]⁺ 353.

*Step 4:* To a 20 mL vial, the *tert*-butyl (*E*)-3-(7-cyano-6-fluoronaphthalen-2-yl)but-2-enoate (40.5 mg, 0.130 mmol), NaN₃ (9.3 mg, 0.14 mmol), and DMSO (1 mL) was added. The mixture was stirred and heated at 100°C for 2h. The mixture was diluted in 1:2 EtOAc/Et₂O (12 mL) and filtered through Celite, rinsing with Et₂O. The filtrate was washed with water (3 x 10 mL). The organic layer was dried over sodium sulfate, filtered, and the solvents of the filtrate were evaporated. The mixture was purified by silica gel chromatography with 0-30% EtOAc in heptane as eluent to afford the *tert*-butyl (*E*)-3-(6-azido-7-cyanonaphthalen-2-yl)but-2-enoate. LRMS [M+H-N₂]⁺ 307.

*Step 5:* To a 20 mL vial, *tert*-butyl (*E*)-3-(6-azido-7-cyanonaphthalen-2-yl)but-2-enoate (13.5 mg, 0.0404 mmol) and formic acid (1 mL) was added. The mixture was stirred and heated at 40°C for 15 min. The solvents were evaporated to afford **A-7** (*E*)-3-(6-azido-7-cyanonaphthalen-2-yl)but-2-enoic acid. LRMS [M-H]⁻ 277.

### Synthesis of A-8:

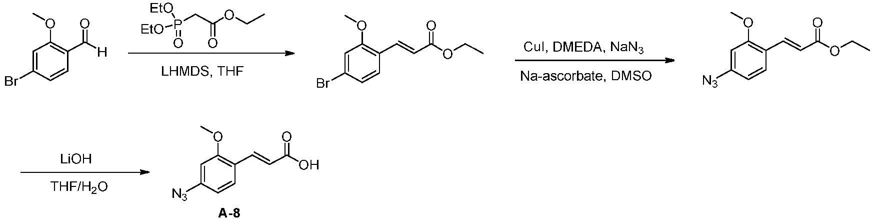

*Step 1:* To a solution of ethyl 2-(diethoxyphosphoryl)acetate (0.60 mL, 3.0 mmol), 1M LHMDS solution in THF (3.3 mL, 3.3 mmol) was added dropwise over 10 min. The mixture was stirred for 30 min at RT before addition of 4-bromo-2-methoxybenzaldehyde (645 mg, 3.00 mmol) in one portion. The reaction was then stirred at RT for 16h and quenched by addition of sat. aq. NH₄Cl (20 mL). The quenched reaction was extracted with EtOAc (30 x 3 mL). The combined organic layer was dried over Na₂SO₄ and concentrated in vacuo to afford the crude. Ethyl (*E*)-3-(4-bromo-3-methoxyphenyl)acrylate was isolated using silica gel chromatography. LRMS [M+H]⁺285.

*Step 2:* Ethyl (*E*)-3-(4-bromo-3-methoxyphenyl)acrylate (285 mg, 1.00 mmol,), CuI (9.5 mg, 0.050 mmol), and Na-ascorbate (20 mg, 0.10 mmol) were charged into a vial purged with N₂. To the mixture, DMSO (5 mL) and DMEDA (17 µL, 0.15 mmol) was added. The mixture was then stirred at RT for 15 min before NaN₃ (98 mg, 1.5 mmol) was added. The reaction was heated at 100°C for 16h. The reaction was then stirred at RT for 16h and quenched by addition of sat. aq. NH₄Cl (20 mL). The quenched reaction was extracted with EtOAc (50 x 3 mL). The combined organic layer was dried over Na₂SO₄ and concentrated in vacuo to afford the crude. Ethyl (*E*)-3-(4-azido-2-methoxyphenyl)acrylate was isolated using silica gel chromatography. LRMS [M+H]⁺ 248.

*Step 3:* To a solution of ethyl (*E*)-3-(4-azido-2-methoxyphenyl)acrylate (170 mg, 0.690 mmol) in THF (4 mL), LiOH (32.0 mg, 1.38 mmol) pre-dissolved in H₂O (2 mL) was added. The reaction mixture was stirred at RT for 3h. The reaction was concentrated in vacuo and diluted with H₂O (20 mL). The aqueous suspension was adjusted to pH 4 and extracted with EtOAc (20 x 3 mL). The combined organic layer was washed with H₂O (30 mL) and brine (30 mL), dried over Na₂SO₄, and concentrated in vacuo to afford the crude. **A-8** was isolated using silica gel chromatography. LRMS [M-H]⁻ 218.

### Synthesis of A-9

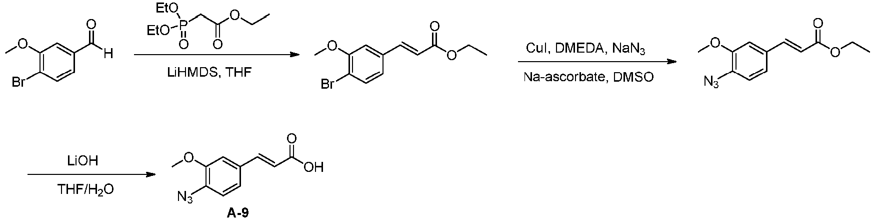

In analogy to the synthesis of A-8, 4-bromo-3-methoxybenzaldehyde was converted in three steps to A-9. LRMS [M-H]⁻ 218.

### Synthesis of A-10

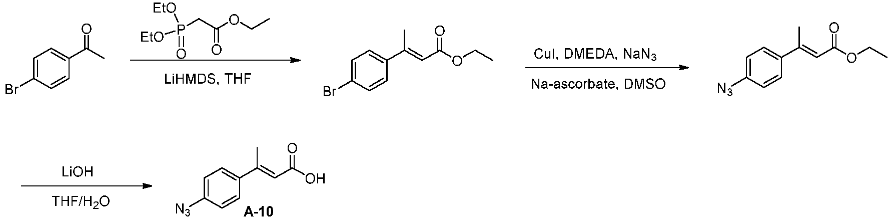

In analogy to the synthesis of **A-8**, 1-(4-bromophenyl)ethan-1-onewas converted in three steps to A-10. LRMS [M-H]⁻ 202.

### Synthesis of A-11

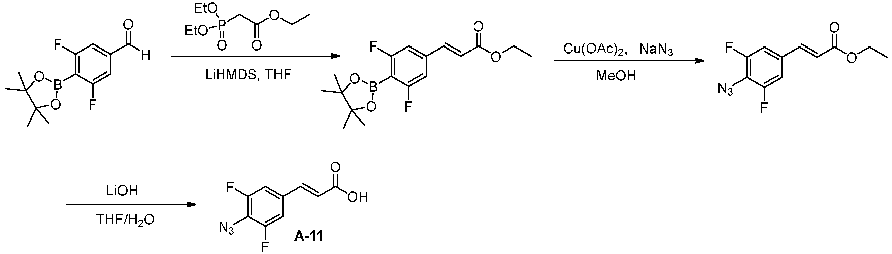

*Step 1:* To a solution of ethyl 2-(diethoxyphosphoryl)acetate (0.32 mL, 1.7 mmol), 1M LHMDS solution in THF (1.7 mL, 1.7 mmol) was added dropwise over 10 min. The mixture was stirred for 30 min at RT before addition of 3,5-difluoro-4-(4,4,5,5-tetramethyl-1,3,2-dioxaborolan-2-yl)benzaldehyde (402 mg, 1.50 mmol) in one portion. The reaction was then stirred at RT for 16h and quenched by addition of sat. aq. NH₄Cl (20 mL). The quenched reaction was extracted with EtOAc (30 x 3 mL). The combined organic layer was dried over Na₂SO₄, concentrated in vacuo to afford the crude. Ethyl (*E*)-₃-(3,5-difluoro-4-(4,4,5,5-tetramethyl-1,3,2-dioxaborolan-2-yl)phenyl)acrylate was isolated using silica gel chromatography. LRMS [M+H]⁺ 339.

*Step 2:* To a solution of ethyl (*E*)-3-(3,5-difluoro-4-(4,4,5,5-tetramethyl-1,3,2-dioxaborolan-2-yl)phenyl)acrylate (73 mg, 0.22 mmol) in MeOH (4 mL), Cu(OAc)₂ (4.0 mg, 0.022 mmol) and NaN₃ (14 mg, 0.22 mmol) was added. The reaction was heated at 60°C for 30 min. The reaction was diluted with EtOAc (50 mL) then quenched by addition of sat. aq. NH₄Cl (10 mL). The aqueous layer was extracted with EtOAc (10 x 3 mL). The combined organic layer was washed with H₂O (30 mL) and brine (30 mL), dried over Na₂SO₄, and concentrated in vacuo. The desired product was purified by silica gel chromatography to afford ethyl (E)-3-(4-azido-3,5-difluorophenyl)acrylate. LRMS [M+H]⁺ 254.

*Step 3:* To a solution of ethyl (E)-3-(4-azido-3,5-difluorophenyl)acrylate (30 mg, 0.12 mmol,) in THF (4 mL), LiOH (80 mg, 2.0 mmol) pre-dissolved in H₂O (2 mL) was added. The reaction mixture was stirred at RT for 3h. The reaction was concentrated in vacuo and diluted with H₂O (20 mL). The aqueous suspension was adjusted to pH 4 and extracted with EtOAc (3 x 20 mL). The combined organic layer was washed with H₂O (30 mL) and brine (30 mL), dried over Na₂SO₄, and concentrated in vacuo to afford the crude. A-11 was isolated using silica gel chromatography. LRMS [M-H]⁻ 224.

### Synthesis of A-12

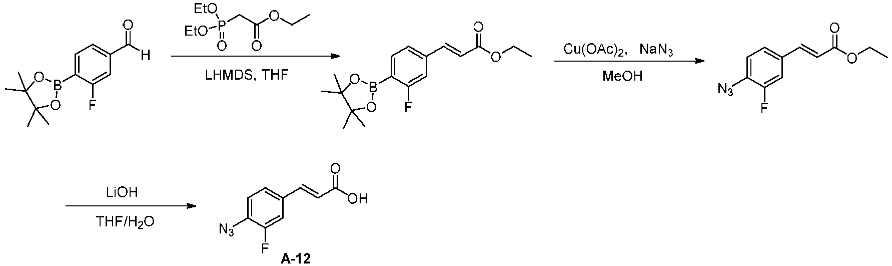

In analogy to the synthesis of A-11, 3-fluoro-4-(4,4,5,5-tetramethyl-1,3,2-dioxaborolan-2-yl)benzaldehyde was converted in three steps to A-12. LRMS [M-H]⁻ 206.

### Synthesis of A-13

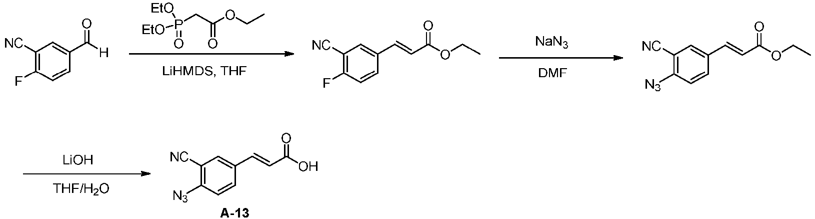

*Step 1:* To a solution of ethyl 2-(diethoxyphosphoryl)acetate (0.57 mL, 2.9 mmol), 1M LHMDS solution in THF (3.1 mL, 3.1 mmol) was added dropwise over 10 min. The mixture was stirred for 30 min at RT before addition of 2-fluoro-5-formylbenzonitrile (425 mg, 2.85 mmol) in one portion. The reaction was then stirred at RT for 16h and quenched by addition of sat. aq. NH₄Cl (20 mL). The quenched reaction was extracted with EtOAc (30 x 3 mL). The combined organic layer was dried over Na₂SO₄ and concentrated in vacuo to afford the crude. Ethyl (E)-3-(3-cyano-4-fluorophenyl)acrylate was isolated using silica gel chromatography. LRMS [M+H]⁺ 220.

*Step 2:* To a solution of Ethyl (*E*)-3-(3-cyano-4-fluorophenyl)acrylate (149 mg, 0.68 mmol) in DMF (3 mL), NaN₃ (66 mg, 1.0 mmol) was added in one portion. The mixture was heated at 70°C overnight. The reaction was cooled down, diluted with EtOAc (50 mL), and poured onto crushed ice. After partitioning in a separatory funnel, the aqueous layer was extracted with EtOAc (3 x 20 mL). The combined organic layer was washed with H₂O (50 mL) and brine (50 mL), dried over Na₂SO₄, and concentrated in vacuo. Ethyl (*E*)-3-(4-azido-3-cyanophenyl)acrylate was isolated using silica gel chromatography. LRMS [M + H]⁺ 243.

*Step 3:* To a solution of ethyl (*E*)-3-(4-azido-3-cyanophenyl)acrylate (25 mg, 0.10 mmol) in THF (4 mL), LiOH (40 mg, 1.0 mmol) pre-dissolved in H₂O (2 mL) was added. The reaction mixture was stirred at RT for 3h. The reaction was concentrated in vacuo and diluted with H₂O (20 mL). The aqueous suspension was adjusted to pH 4 and extracted with EtOAc (3 x 20 mL). The combined organic layer was washed with H₂O (30 mL) and brine (30 mL), dried over Na₂SO₄, and concentrated in vacuo to afford the crude. **A-13** was isolated using silica gel chromatography. LRMS [M-H]⁻ 213.

### Synthesis of A-14

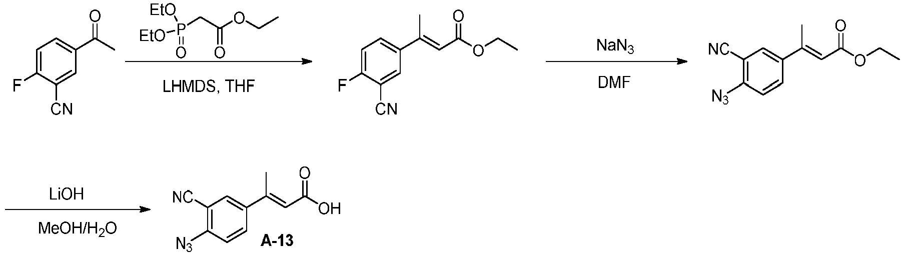

In analogy to the synthesis of **A-13,** 5-acetyl-2-fluorobenzonitrile was converted in three steps to **A-14.** LRMS [M-H]⁻ 227.

### Synthesis of vinyl-extended aryl-azide-biotins

### Synthesis of compound 9575

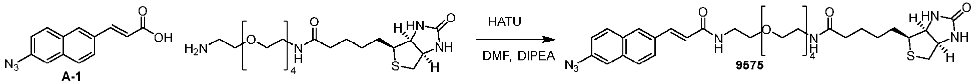

To a 20 mL vial, **A-1,** biotin-PEG-4-amine, DMF (1 mL), and DIPEA (0.161 mL, 0.920 mmol) was added. To the mixture, HATU (69.9 mg, 0.184 mmol) was added. The mixture was purified by RP HPLC (MeCN/water w/ 0.1% TFA) to afford compound **9575.** LRMS [M+H]⁺ 684. ¹H NMR (400 MHz, DMSO-*d*₆) δ 8.22 (t, *J =* 5.7 Hz, 1H), 8.07 (s, 1H), 8.00 (d, *J =* 8.8 Hz, 1H), 7.94 (d, *J* = 8.7 Hz, 1H), 7.83 (t, *J* = 5.7 Hz, 1H), 7.74 (dd, *J* = 8.7, 1.7 Hz, 1H), 7.70 (d, *J* = 2.2 Hz, 1H), 7.57 (d, *J =* 15.7 Hz, 1H), 7.31 (dd, *J =* 8.7, 2.3 Hz, 1H), 6.79 (d, *J =* 15.7 Hz, 1H), 6.42 (s, 1H), 6.36 (s, 1H), 4.30 (dd, *J = 7.8,* 5.0 Hz, 1H), 4.12 (dd, *J =* 7.8, 4.4 Hz, 1H), 3.56 - 3.45 (m, 16H), 3.40 - 3.35 (m, 4H), 3.12 - 3.03 (m, 1H), 2.81 (dd, *J =* 12.5, 5.1 Hz, 1H), 2.57 (d, *J =* 12.4 Hz, 1H), 2.06 (t, *J= 7.4* Hz, 2H), 1.67 - 1.40 (m, 4H), 1.37 - 1.22 (m, 3H).

The compounds in the following table were synthesized in a manner analogous to the synthesis of **9575** from the corresponding aryl azide intermediate (or commercially available aryl azide) and biotin-PEG4-amine.

| **Compound** | **Structure** | **LRMS [M+H]⁺** |
|---|---|---|
| **9582** | 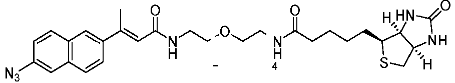 | 698 |
| **9595** | 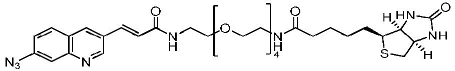 | 685 |
| **9917** | 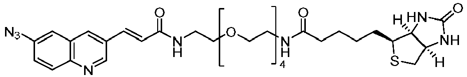 | 685 |
| **9599** | 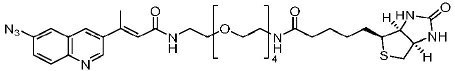 | 699 |
| **9615** | 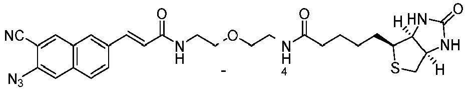 | 709 |
| **9616** | 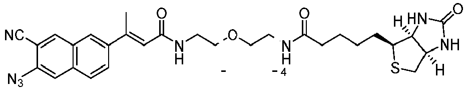 | 723 |
| **9158** | 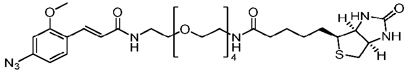 | 664 |
| **9157** | 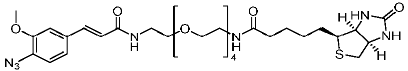 | 664 |
| **9044** | 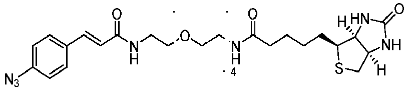 | 634 |
| **9162** | 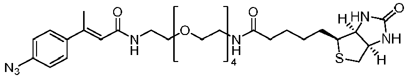 | 648 |
| **9161** | 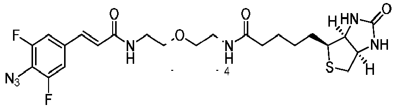 | 670 |
| **9160** | 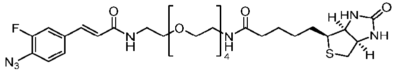 | 652 |
| **9159** | 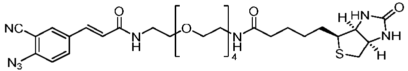 | 659 |
| **9422** | 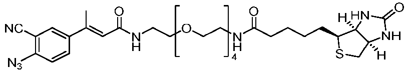 | 673 |

### Example 4

### Synthesis of the Ir catalyst

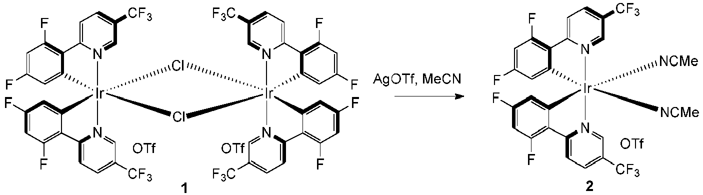

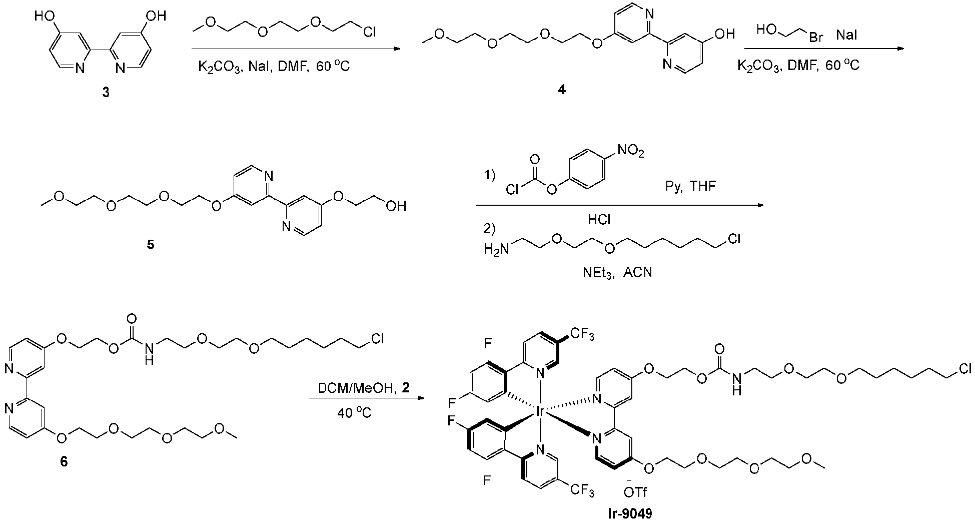

**Intermediate 2:** The bi-Ir-Cl complex 1 (0.1 mmol, 1.0 equiv) was combined with AgOTf (53 mg, 0.2 mmol, 2.0 equiv) in CH₃CN (5 mL). This mixture was stirred at RT overnight in the dark. The resulting suspension was then filtered through celite and concentrated. The residue was redissolved DCM/MeOH (1/1, 10 mL), filtered through celite, and concentrated to yield the intermediate 2 as yellow film that was used without further purification.

**Intermediate 4:** To a solution of **3** (94 mg, 0.5 mmol, 1.0 equiv) in DMF (5 mL), NaI (7.5 mg, 0.05 mmol, 0.1 equiv), 1-chloro-2-(2-(2-methoxyethoxy)etho-xy)ethane (110 mg, 0.6 mmol, 1.2 equiv), and K₂CO₃ (207 mg, 1.5 mmol, 3.0 equiv) was added. The suspension was heated at 60⁰C overnight. After cooling down, the mixture was diluted with EtOAc (50 mL), filtered over Celite, and the filtrate was concentrated in vacuo to afford the crude. The desired product was isolated by silica gel chromatography. ¹H NMR (400 MHz, Methanol-*d*₄) δ 8.63 (d, *J* = 5.9 Hz, 1H), 8.46 (dd, J= 6.7, 2.1 Hz, 1H), 7.90 (s, 1H), 7.85 (s, 1H), 7.32 - 7.23 (m, 1H), 7.20 (d, *J=* 6.7 Hz, 1H), 4.43 (t, *J =* 4.6 Hz, 2H), 3.93 (p, *J* = 2.2 Hz, 2H), 3.76 - 3.47 (m, 8H), 3.35 (s, 3H). LRMS [M + H]⁺ 335.4.

**Intermediate 5:** To a solution of **4** (25 mg, 75 µmol, 1.0 equiv) in DMF (5 mL), bromoethanol (47 mg, 374 µmol, 5.0 equiv), NaI (1.2 mg, 7.5 µmol, 0.1 equiv), and K₂CO₃ (31 mg, 224 µmol, 3.0 equiv) was added. The mixture was stirred at 60⁰C overnight. After cooling down, the mixture was diluted with EtOAc (50 mL), filtered over Celite, and the filtrate was concentrated in vacuo to afford the crude. The desired product was isolated by silica gel chromatography. ¹H NMR (400 MHz, Chloroform-*d*) δ 8.66 - 8.44 (m, 2H), 8.13 - 7.75 (m, 2H), 7.00 - 6.85 (m, 2H), 6.79 (brs, 1H), 4.37 - 4.20 (m, 4H), 4.04 - 3.58 (m, 12H), 3.34 (s, 3H). LRMS [M + H]⁺ 379.4.

**Intermediate 6:** To a solution of **5** (16 mg, 0.04 mmol, 1.0 equiv) in THF (4 mL), pyridine (0.5 mL) and p-nitrophenyl chloroformate (10 mg, 0.05 mmol, 1.2 equiv) was added. The solution was stirred at RT overnight. The reaction was diluted with DCM (10 mL), filtered over Celite, and the filtrate was concentrated in vacuo to afford the crude, which was used in the next step without further purification.

To a solution of the crude from previous step in ACN (2 mL), the chloroalkane amine reactive agent (39 mg, 150 µmol, 3 equiv) and NEt₃ (0.2 mL) was added. The solution was stirred at RT overnight and concentrated onto celite. The desired product was isolated using silica gel chromatography. ¹H NMR (400 MHz, *Methanol-d4*) δ 8.48- 8.46 (m, 2H), 7.95 - 7.74 (m, 2H), 7.17 - 6.92 (m, 2H), 4.47 - 4.25 (m, 6H), 3.95 - 3.42 (m, 20H), 3.35 (s, 3H), 3.29 - 3.17 (m, 2H), 1.77 - 1.50 (m, 2H), 1.63 - 1.50 (m, 2H), 1.47 - 1.21 (m, 4H). LRMS [M + H]⁺ 628.3.

**Ir-9049: Intermediate 2** (14 mg, 15 µmol, 1.0 equiv) and 6 (9.4 mg, 15 µmol, 1.0 equiv) was dissolved in DCM/MeOH (1/1, 2 mL). The solution was stirred at RT overnight. The desired product was isolated by silica column with DCM/MeOH as eluent. ¹H NMR (400 MHz, Methanol-*d*₄) δ 8.58 (d, *J =* 8.9 Hz, 2H), 8.39 (s, 2H), 8.33 (d, *J =* 8.9 Hz, 2H), 7.84 (d, *J =* 6.9 Hz, 4H), 7.27 (s, 2H), 6.81 (t, *J =* 10.9 Hz, 2H), 5.79 (d, *J =* 8.0 Hz, 2H), 4.47 - 4.25 (m, 4H), 3.92- 3.78 (m, 2H), 3.75 - 3.58 (m, 20H), 3.32 (s, 3H), 1.78 - 1.70 (m, 2H), 1.63 - 1.50 (m, 2H), 1.47 - 1.37 (m, 4H). LRMS [M + H]⁺ 1336.7.

## Claims

1. A compound of formula (I): 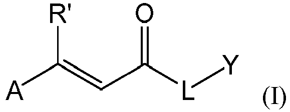 or a salt thereof, wherein:
A is selected from: 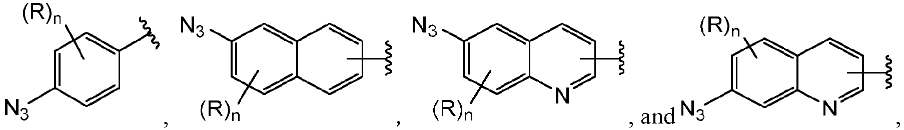 wherein:
each n is independently 1, 2, 3, or 4; and
each R is independently selected from hydrogen, halo, C₁-C₄ alkyl, C₂-C₄ alkenyl, hydroxy, mercapto, amino, cyano, C₁-C₄-alkoxy, halo-C₁-C₄-alkyl, hydroxy-C₁-C₄-alkyl, amino-C₁-C₄-alkyl, mercapto-C₁-C₄-alkyl, cyano-C₁-C₄-alkyl, - C(O)-C₁-C₄-alkyl, -C(O)OH, and -C(O)NH₂;
R' is hydrogen or C₁-C₄ alkyl;
L is a linker; and
Y is a functional moiety selected from:
(a) biotin;
(b) a haloalkane group;
(c) a fluorescent functional group selected from a xanthene, a cyanine, a naphthalene, an oxadiazole, a pyrene, an oxazine, an acridine, an arylmethine, a tetrapyrrole, a coumarin, a squaraine, and a boron-dipyrromethene; and
(d) a reactive functional group comprising an azide, alkyne, alkene, or 1,2,4,5-tetrazinyl moiety.

2. The compound of claim 1, or a salt thereof, wherein A is: 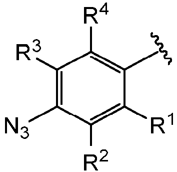 wherein:
R¹, R², R³, and R⁴ are each independently selected from hydrogen, halo, hydroxy, cyano, and C₁-C₄ alkoxy;
optionally wherein:
R¹ is hydrogen, hydroxy, or C₁-C₄ alkoxy;
R² is hydrogen, halo, cyano, or C₁-C₄ alkoxy;
R³ is hydrogen or halo; and
R⁴ is hydrogen or halo.

3. The compound of claim 1, or a salt thereof, wherein:
(i) A is: 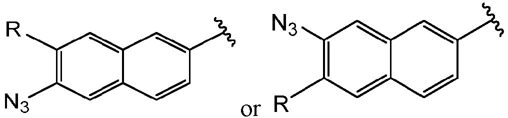 wherein each R is independently selected from hydrogen, halo, cyano, and C₁-C₄ alkoxy;
(ii) A is: 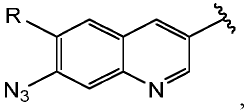 wherein:
R is selected from hydrogen, halo, cyano, and C₁-C₄ alkoxy; or
(iii) A is: 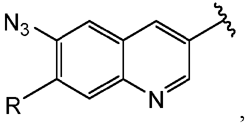 wherein:
R is selected from hydrogen, halo, cyano, and C₁-C₄ alkoxy.

4. The compound of claim 1, or a salt thereof, wherein A has a formula selected from: 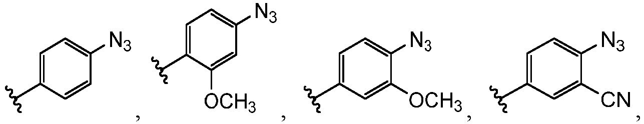 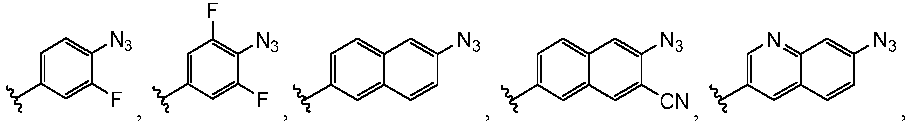 and

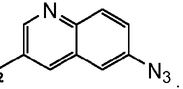

5. The compound of any one of claims 1-4, or a salt thereof, wherein R' is selected from hydrogen and methyl.

6. The compound of any one of claims 1-5, or a salt thereof, wherein the linker comprises one or more moieties selected from straight or branched chain alkylene, ether (-O-), amine (-NH-), ester (-C(O)O-), amide (-C(O)NH-), carbamate (-NHC(O)O-), urea (-NHC(O)NH-), and phenylene groups.

7. The compound of any one of claims 1-6, or a salt thereof, wherein the linker has a formula:
-NHCH₂CH₂(OCH₂CH₂)ₙNH-
wherein n is 1, 2, 3, 4, 5, 6, 7, or 8.

8. The compound of any one of claims 1-7, or a salt thereof, wherein Y has a formula: 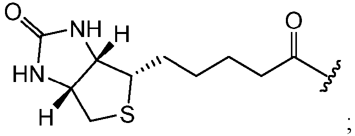 or wherein Y has a formula -(CH₂)ₙ-X, wherein n is 2, 3, 4, 5, 6, 7, 8, 9, 10, 11, or 12, and X is a halogen.

9. The compound of claim 1, selected from the group consisting of: 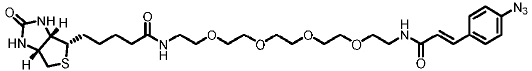 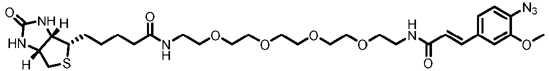 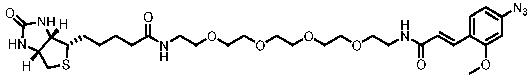 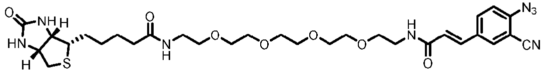 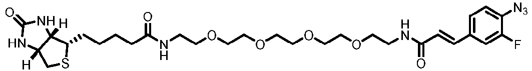 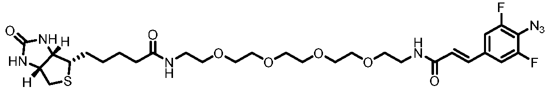 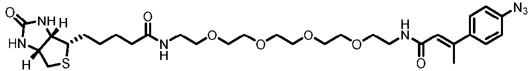 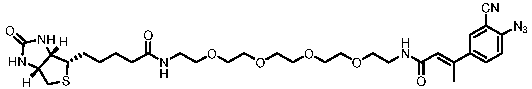 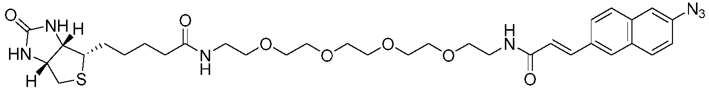 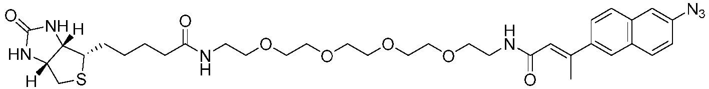 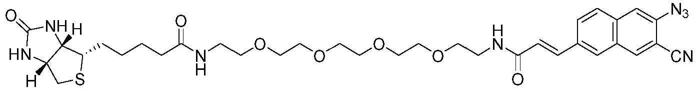 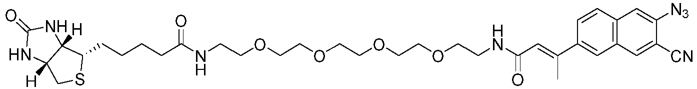 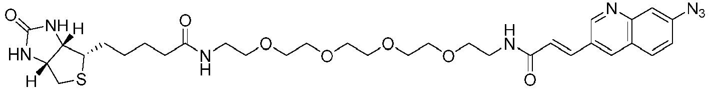 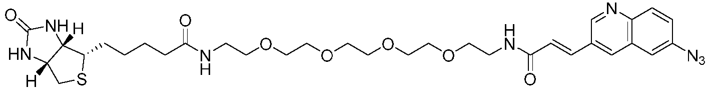 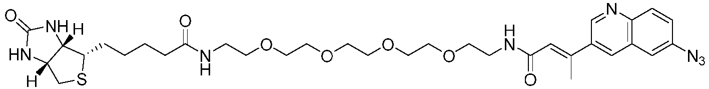 and salts thereof.

10. A system for photocatalytic labeling of a biomolecule, comprising:
(a) a compound of any one of claims 1-9; and
(b) a photocatalyst.

11. The system of claim 10, wherein the photocatalyst has a structure 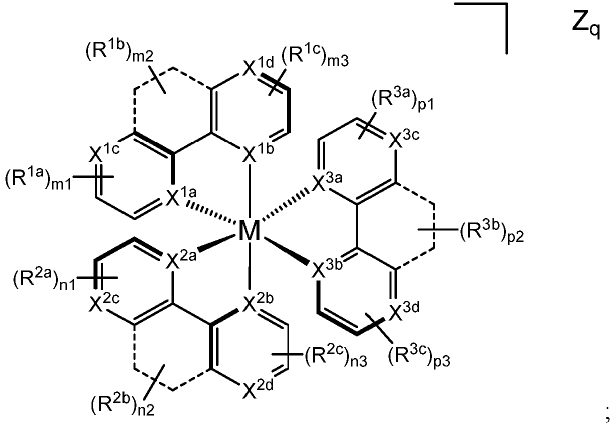 wherein:
each set of dashed lines (------) represents the presence or absence of a fused 6-membered ring;
M is a transition metal;
m1, m2, m3, n1, n2, n3, p1, p2, and p3 are each independently 0, 1, or 2;
R^{1a}, R^{1b}, R^{1c}, R^{2a}, R^{2b}, R^{2c}, R^{3a}, R^{3b}, and R^{3c} are each independently selected from halo, alkyl, haloalkyl, amino, and heteroalkyl;
X^{1a}, X^{1b}, X^{2a}, X^{2b}, X^{3a}, and X^{3b} are each independently selected from N and C, wherein at least one of X^{1a} and X^{1b} is N, at least one of X^{2a} and X^{2b} is N, and at least one of X^{3a} and X^{3b} is N;
X^{1c}, X^{1d}, X^{2c}, X^{2d}, X^{3c}, and X^{3d} are each independently selected from CH and N; A is an anion; and
q is 0, 1, or 2;
optionally wherein the transition metal is selected from Ru and Ir.

12. The system of claim 10, wherein:
(i) the photocatalyst is an iridium photocatalyst selected from: 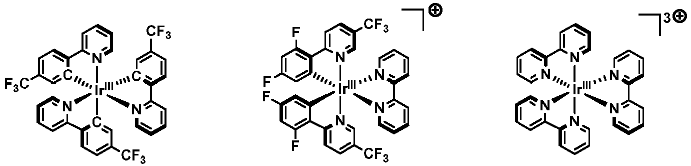 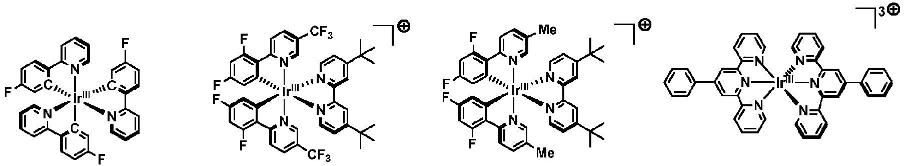 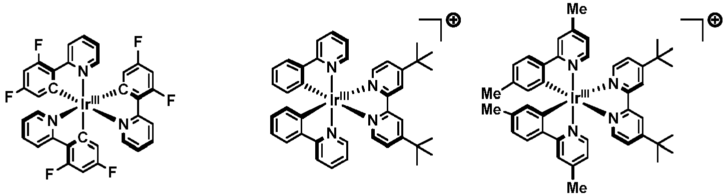 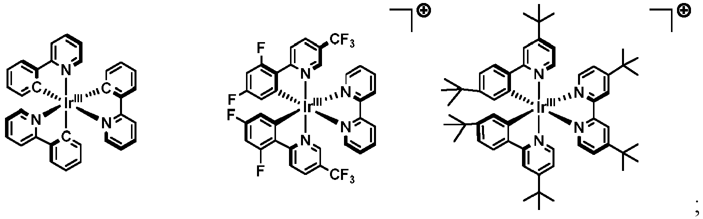 or
(ii) the photocatalyst is a ruthenium-based photocatalyst selected from:

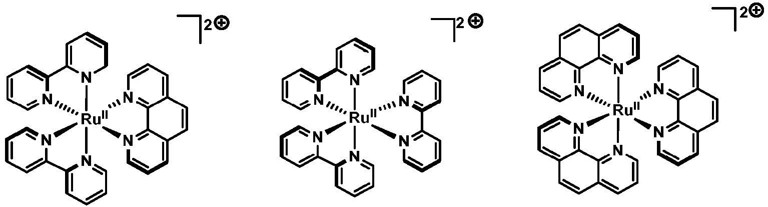

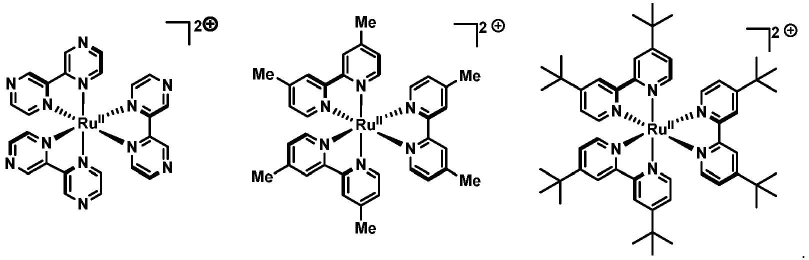

13. The system of claim 11, wherein the photocatalyst is of the formula:

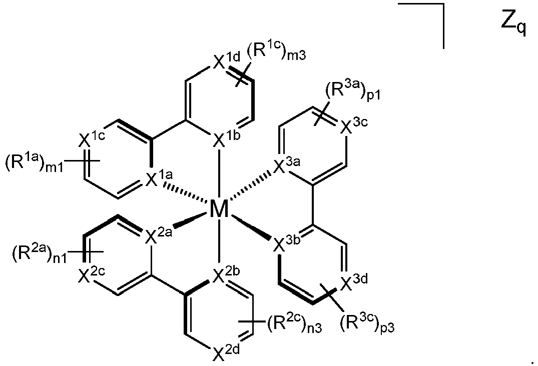

14. A method of labeling a biomolecule in a sample, comprising:
(a) contacting the sample with a compound of any one of claims 1-9; and
(b) exposing the sample to light, optionally wherein: (i) the light is selected from ultraviolet light and visible light; (ii) the light is visible light from a light-emitting diode; or (iii) the light is bioluminescent light.

15. The method of claim 14, further comprising contacting the sample with a photocatalyst in step (a).

## Patentansprüche

1. Verbindung der Formel (I): 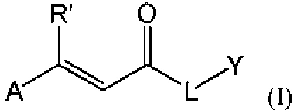 oder ein Salz davon, wobei:
A ausgewählt ist aus: 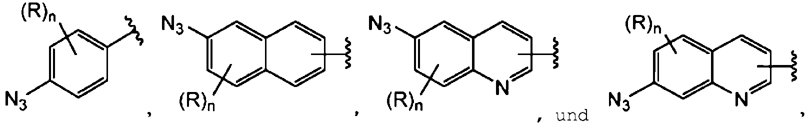
wobei:
jedes n unabhängig 1, 2, 3 oder 4 ist; und
jedes R unabhängig voneinander aus Wasserstoff, Halogen, C₁-C₄-Alkyl, C₂-C₄-Alkenyl, Hydroxy, Mercapto, Amino, Cyano, C₁-C₄-Alkoxy, Halogen-C₁-C₄-Alkyl, Hydroxy-₁-C₄-Alkyl, Amino-C₁-C₄-Alkyl, Mercapto-C₁-C₄-Alkyl, Cyano-C₁-C₄-Alkyl, -C(O)-C₁-C₄-Alkyl, -C(O)OH und -C(O)NH₂ ausgewählt ist;
R' Wasserstoff oder C₁-C₄-Alkyl ist;
L ein Linker ist; und
Y eine funktionelle Gruppe ist, ausgewählt aus:
(a) Biotin;
(b) einer Halogenalkangruppe;
(c) einer fluoreszierenden funktionellen Gruppe, ausgewählt aus einem Xanthen, einem Cyanin, einem Naphthalin, einem Oxadiazol, einem Pyren, einem Oxazin, einem Acridin, einem Arylmethin, einem Tetrapyrrol, einem Cumarin, einem Squaraine und einem Bordon-Dipyrromethene; und
(d) einer reaktiven funktionellen Gruppe, umfassend eine Azid-Alkin-, Alken- oder 1,2,4,5-Tetrazinylgruppe.

2. Verbindung nach Anspruch 1 oder ein Salz davon, wobei A Folgendes ist: 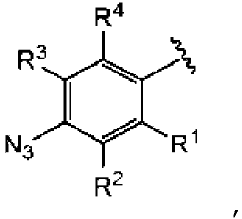 wobei:
R¹, R², R³ und R⁴ jeweils unabhängig voneinander aus Wasserstoff, Halogen, Hydroxy, Cyano und C₁-C₄-Alkoxy ausgewählt sind;
optional wobei:
R¹ Wasserstoff, Hydroxy oder C₁-C₄-Alkoxy ist;
R² Wasserstoff, Halogen, Cyano oder C₁-C₄-Alkoxy ist;
R³ Wasserstoff oder Halogen ist; und
R⁴ Wasserstoff oder Halogen ist.

3. Verbindung nach Anspruch 1 oder ein Salz davon, wobei:
(i) A Folgendes ist: 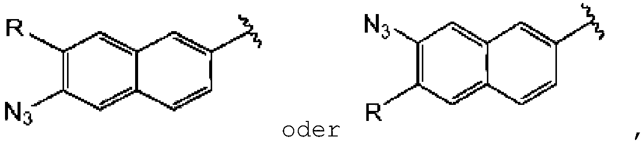 wobei jedes R unabhängig voneinander aus Wasserstoff, Halogen, Cyano und C₁-C₄-Alkoxy ausgewählt ist;
(ii) A Folgendes ist: 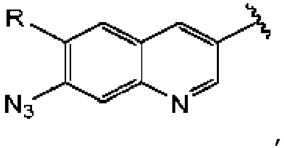 wobei:
R aus Wasserstoff, Halogen, Cyano und C₁-C₄-Alkoxy ausgewählt ist; oder
(iii) A Folgendes ist: 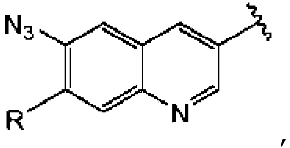 wobei:
R aus Wasserstoff, Halogen, Cyano und C₁-C₄-Alkoxy ausgewählt ist.

4. Verbindung nach Anspruch 1 oder ein Salz davon, wobei A eine Formel aufweist, die ausgewählt ist aus: 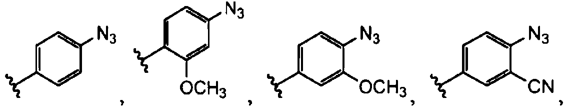 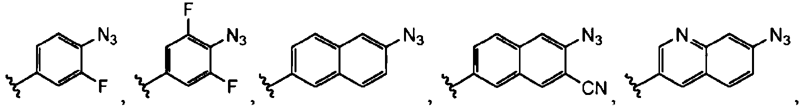 und

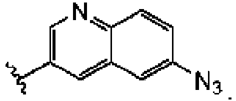

5. Verbindung nach einem der Ansprüche 1-4 oder ein Salz davon, wobei R' aus Wasserstoff und Methyl ausgewählt ist.

6. Verbindung nach einem der Ansprüche 1-5 oder ein Salz davon, wobei der Linker eine oder mehrere Einheiten umfasst, die aus geradkettigen oder verzweigten Alkylen-, Ether- (-O-), Amin- (-NH-), Ester- (-C(O)O-), Amid- (-C(O)NH-), Carbamat (-NHC(O)O-), Harnstoff (-NHC(O)NH-) und Phenylengruppen ausgewählt ist.

7. Verbindung nach einem der Ansprüche 1-6 oder ein Salz davon, wobei der Linker die folgende Formel aufweist:
-NHCH₂CH₂(OCH₂CH₂)ₙNH-
wobei n 1, 2, 3, 4, 5, 6, 7 oder 8 ist.

8. Verbindung nach einem der Ansprüche 1-7 oder ein Salz davon, wobei Y die folgende Formel aufweist: 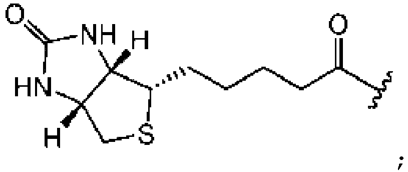 oder wobei Y die folgende Formel aufweist:
-(CH₂)ₙ-X, wobei n 2, 3, 4, 5, 6, 7, 8, 9, 10, 11 oder 12 ist und X ein Halogen ist.

9. Verbindung nach Anspruch 1, ausgewählt aus der Gruppe bestehend aus: 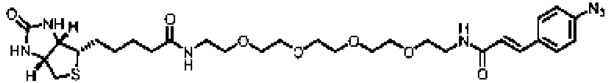 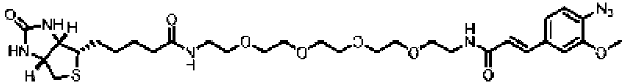 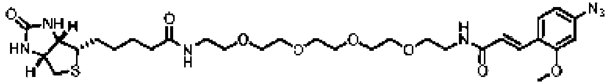 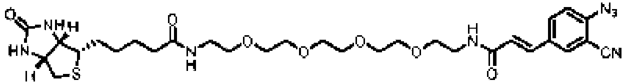 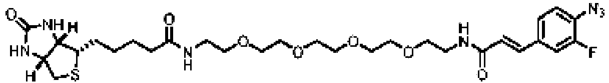 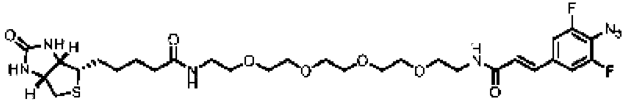 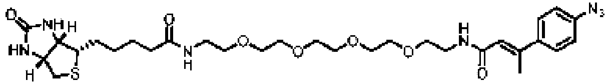 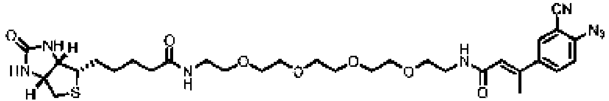 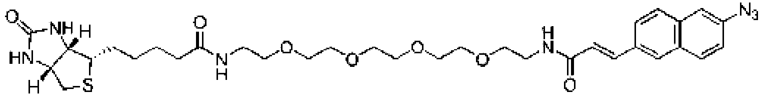 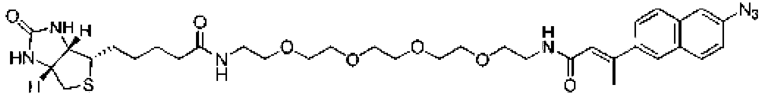 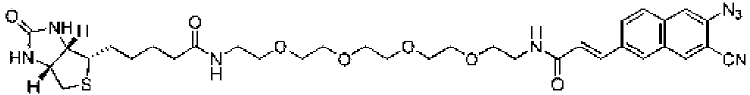 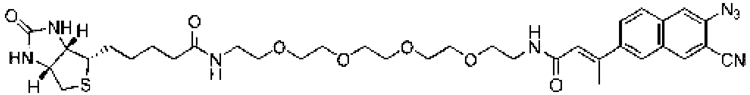 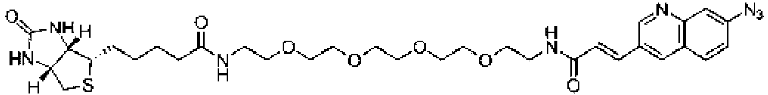 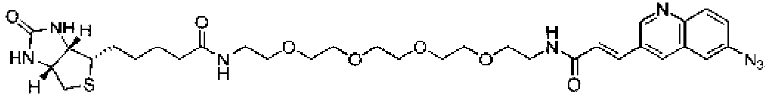 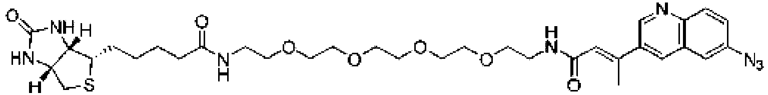 und deren Salzen.

10. System zur photokatalytischen Markierung eines Biomoleküls, umfassend:
(a) eine Verbindung nach einem der Ansprüche 1-9; und
(b) einen Photokatalysator.

11. System nach Anspruch 10, wobei der Photokatalysator folgende Struktur aufweist 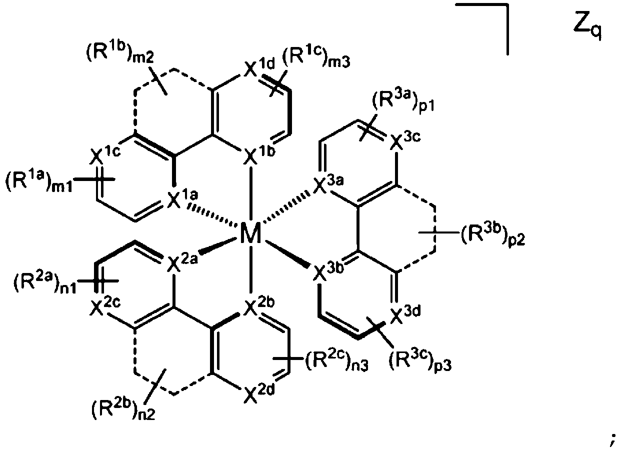 wobei:
jeder Satz gestrichelter Linien (------) das Vorhandensein oder Fehlen eines fusionierten 6-gliedrigen Elements darstellt;
M ein Übergangsmetall ist;
ml, m2, m3, n1, n2, n3, p1, p2 und p3 jeweils unabhängig voneinander 0, 1 oder 2 sind;
R^{1a}, R^{1b}, R^{1c}, R^{2a}, R^{2b}, R^{2c}, R^{3a}, R^{3b} und R^{3c} jeweils unabhängig voneinander aus Halogen, Alkyl, Halogenalkyl, Amino und Heteroalkyl ausgewählt sind;
X^{1a}, X^{1b}, X^{2a}, X^{2b}, X^{3a} und X^{3b} jeweils unabhängig voneinander aus N und C ausgewählt sind, wobei mindestens eines von X^{1a} und X^{1b} N ist, mindestens eines von X^{2a} und
X^{2b} N ist und mindestens eines von X^{3a} und X^{3b} N ist;
X^{1c}, X^{1d}, X^{2c}, X^{2d}, X^{3c} und X^{3d} jeweils unabhängig voneinander aus CH und N ausgewählt sind;
A ein Anion ist; und
q 0, 1 oder 2 ist;
das Übergangsmetall optional aus Ru und Ir ausgewählt wird.

12. System nach Anspruch 10, wobei:
(i) der Photokatalysator ein Iridium-Photokatalysator ist, ausgewählt aus: 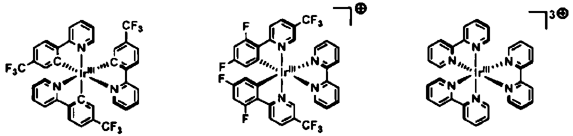 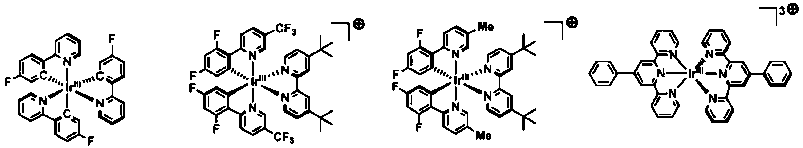 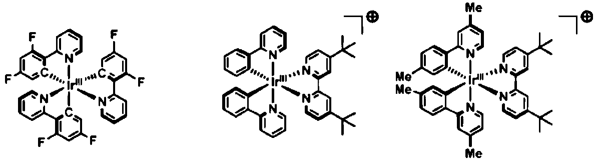 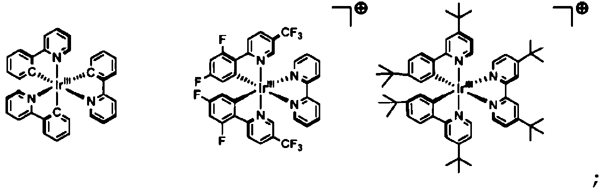 oder
(ii) der Photokatalysator ein Photokatalysator auf Rutheniumbasis ist, ausgewählt aus:

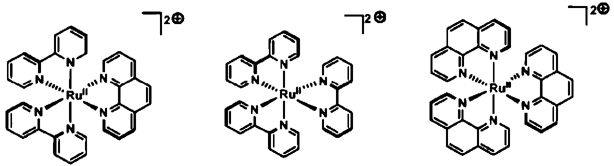

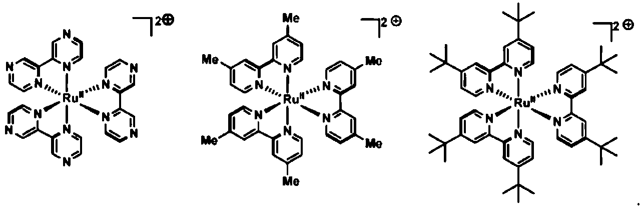

13. System nach Anspruch 11, wobei der Photokatalysator die folgende Formel aufweist:

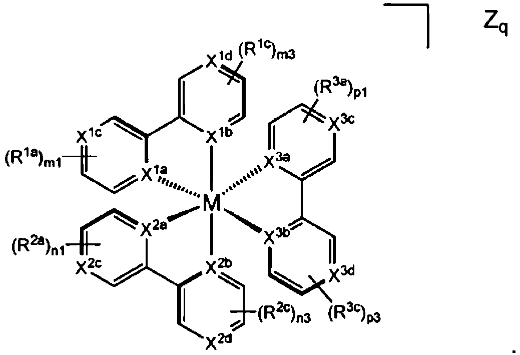

14. Verfahren zum Markieren eines Biomoleküls in einer Probe, umfassend:
(a) Inkontaktbringen der Probe mit einer Verbindung nach einem der Ansprüche 1-9; und
(b) Aussetzen der Probe gegenüber Licht, wobei optional: (i) das Licht aus ultraviolettem Licht und sichtbarem Licht ausgewählt ist; (ii) das Licht sichtbares Licht von einer Leuchtdiode ist; oder (iii) das Licht biolumineszentes Licht ist.

15. Verfahren nach Anspruch 14, ferner umfassend das Inkontaktbringen der Probe mit einem Photokatalysator in Schritt (a).

## Revendications

1. Composé de la formule (I) : 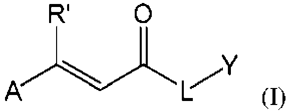 ou sel de celui-ci, dans lequel :
A est sélectionné parmi : 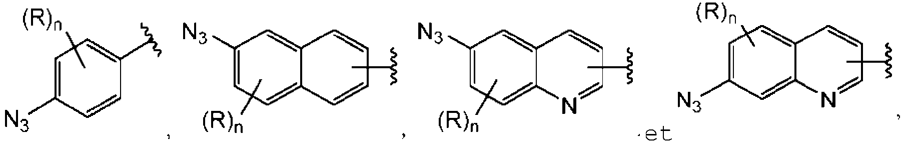
dans lequel :
chaque n vaut indépendamment 1, 2, 3 ou 4 ; et
chaque R est sélectionné indépendamment parmi l'hydrogène, le halo, l'alkyle C₁-C₄ l'alcényle C₂-C₄, l'hydroxy, le mercapto, l'amino, le cyano, l'alcoxy C₁-C₄, le halo-alkyle C₁-C₄, l'hydroxy-alkyle C₁-C₄, l'amino-alkyle C₁-C₄, le mercapto-alkyle C₁-C₄, le cyano-alkyle C₁-C₄, -C(O)-alkyle C₁-C₄, -C(O)OH et -C(O)NH₂ ;
R' est un hydrogène ou un alkyle en C₁-C₄ ;
L est un connecteur ; et
Y est un fragment fonctionnel sélectionné parmi :
(a) la biotine ;
(b) un groupe d'haloalcanes ;
(c) un groupe fonctionnel fluorescent choisi parmi un xanthène, une cyanine, un naphtalène, un oxadiazole, un pyrène, une oxazine, une acridine, une arylméthine, un tétrapyrrole, une coumarine, une squaraïne et un boron-dipyrrométhène ; et
(d) un groupe fonctionnel réactif comprenant un fragment azide, alcyne, alcène ou 1,2,4,5-tétrazinyle.

2. Composé selon la revendication 1, ou sel de celui-ci, dans lequel A est : 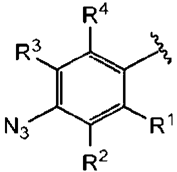 dans lequel :
R¹, R², R³, et R⁴ sont chacun sélectionnés indépendamment parmi l'hydrogène, le halo, l'hydroxy, le cyano et
l'alcoxy C₁-C₄ ;
éventuellement dans lequel :
R¹ est un hydrogène, un hydroxy ou un alcoxy en C₁-C₄ ;
R² est un hydrogène, un halo, un cyano ou un alcoxy en C₁-C₄ ;
R³ représente l'hydrogène ou un halo ; et
R⁴ représente l'hydrogène ou un halo.

3. Composé selon la revendication 1, ou sel de celui-ci, dans lequel :
(i) A est : 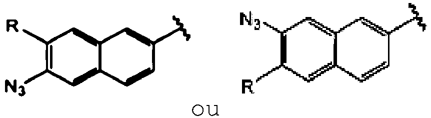 dans lequel chaque R est sélectionné indépendamment parmi l'hydrogène, le halo, le cyano et l'alcoxy en C₁-C₄ ;
(ii) A est : 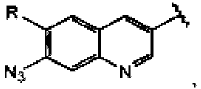 dans lequel :
R est choisi parmi l'hydrogène, le halo, le cyano et l'alcoxy en C₁-C₄ ; ou
(iii) A est : 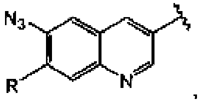 dans lequel :
R est choisi parmi l'hydrogène, le halo, le cyano et l'alcoxy en C₁-C₄.

4. Composé selon la revendication 1, ou sel de celui-ci, dans lequel A a une formule choisie parmi : 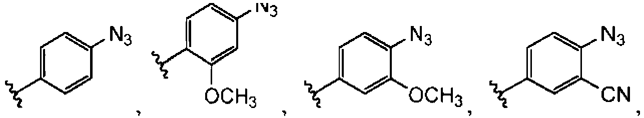 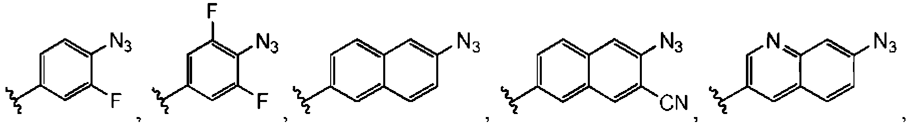 et

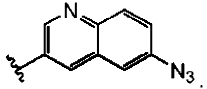

5. Composé selon l'une quelconque des revendications 1 à 4, ou sel de celui-ci, dans lequel R' est choisi parmi l'hydrogène et le méthyle.

6. Composé selon l'une quelconque des revendications 1 à 5, ou sel de celui-ci, dans lequel le lieur comprend un ou plusieurs fragments choisis parmi les groupes alkylène à chaîne droite ou ramifiée, éther (-O-), amine (-NH-), ester (-C(O)O-), amide (-C(O)NH-), carbamate (-NHC(O)O-), urée (-NHC(O)NH-) et phénylène.

7. Composé selon l'une quelconque des revendications 1 à 6, ou sel de celui-ci, dans lequel le lieur a une formule :
-NHCH₂CH₂(OCH₂CH₂)ₙNH-
dans lequel n est 1, 2, 3, 4, 5, 6, 7, ou 8.

8. Composé selon l'une quelconque des revendications 1 à 7, ou sel de celui-ci, dans lequel Y a une formule : 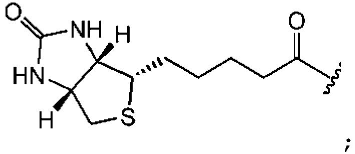 ou dans lequel Y a une formule -(CH₂)ₙ-X, dans lequel n est 2, 3, 4, 5, 6, 7, 8, 9, 10, 11 ou 12, et X est un halogène.

9. Composé selon la revendication 1, choisi dans le groupe constitué de : 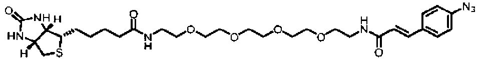 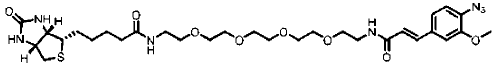 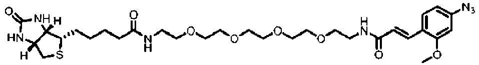 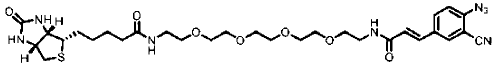 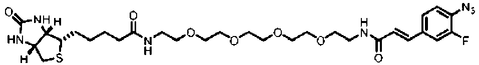 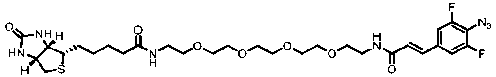 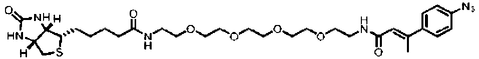 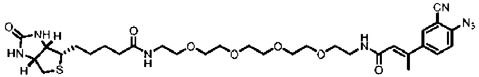 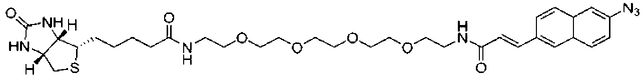 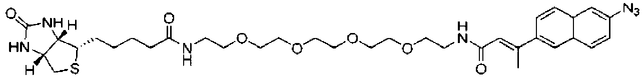 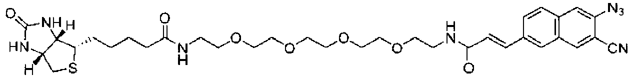 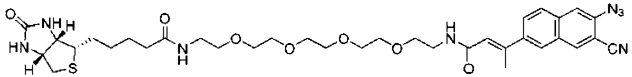 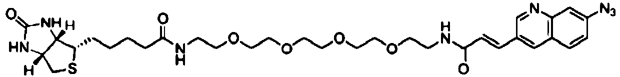 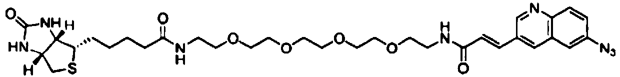 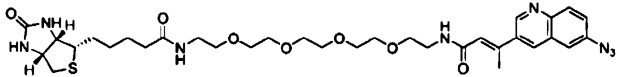 et sels de ceux-ci.

10. Système de marquage photocatalytique d'une biomolécule, comprenant :
(a) un composé selon l'une quelconque des revendications 1 à 9 ; et
(b) un photocatalyseur.

11. Système selon la revendication 10, dans lequel le photocatalyseur possède une structure 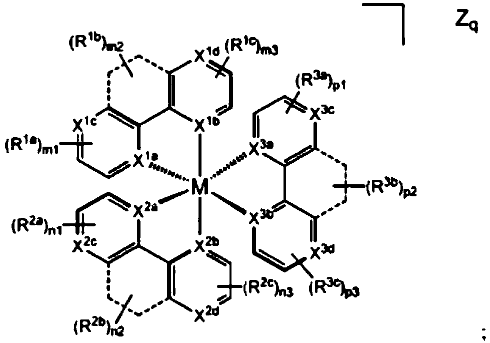 dans lequel :
chaque ensemble de lignes pointillées (------) représente la présence ou l'absence d'un cycle à 6 chaînons fusionné ;
M est un métal de transition ;
m1, m2, m3, n1, n2, n3, p1, p2 et p3 sont chacun indépendamment 0, 1 ou 2 ;
R^{1a}, R^{1b}, R^{1c}, R^{2a}, R^{2b}, R^{2c}, R^{3a}, R^{3b}, et R^{3c} sont chacun sélectionnés indépendamment parmi le halo, l'alkyl, le haloalkyl, l'amino et l'hétéroalkyle ;
X^{1a}, X^{1b}, X^{2a}, X^{2b}, X^{3a}, et X^{3b} sont chacun sélectionnés indépendamment parmi N et C, dans lequel au moins un de X^{1a} et X^{1b} est N, au moins un de X^{2a} et X^{2b} est N, et au moins un de X^{3a} et X^{3b} est N ;
X^{1c}, X^{1d}, X^{2c}, X^{2d}, X^{3c}, et X^{3d} sont chacun sélectionnés indépendamment à partir de CH et N ;
A est un anion ; et
q est 0, 1 ou 2 ;
éventuellement, dans lequel le métal de transition est choisi parmi Ru et Ir.

12. Système selon la revendication 10, dans lequel :
(i) le photocatalyseur est un photocatalyseur à base d'iridium sélectionné parmi : 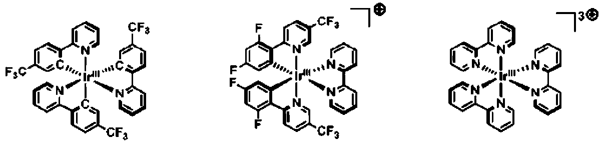 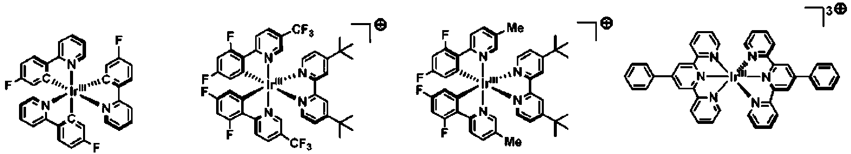 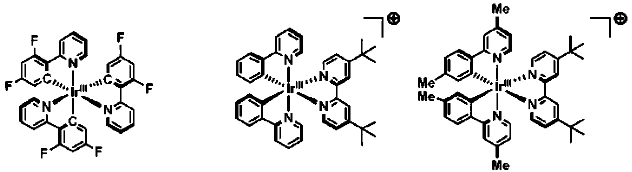 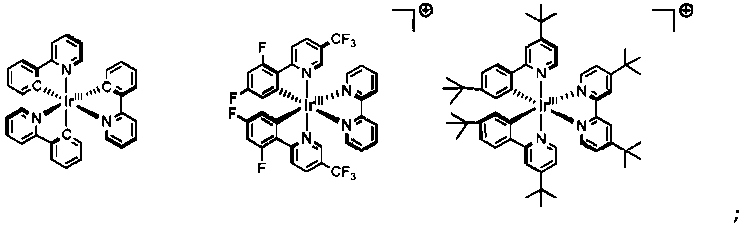 ou
(ii) le photocatalyseur est un photocatalyseur à base de ruthénium sélectionné parmi :

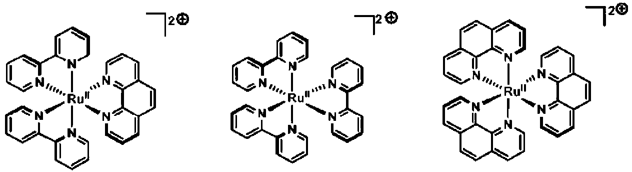

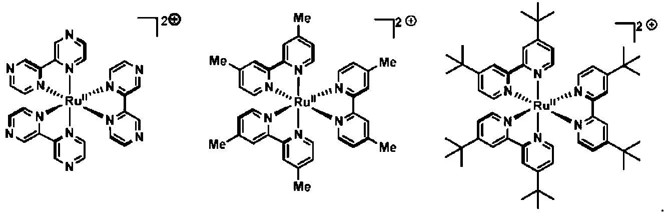

13. Système selon la revendication 11, dans lequel le photocatalyseur a la formule suivante :

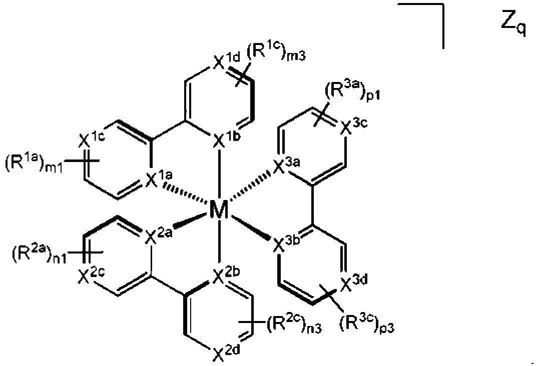

14. Procédé de marquage d'une biomolécule dans un échantillon, comprenant :
(a) la mise en contact de l'échantillon avec un composé selon l'une quelconque des revendications 1 à 9 ; et
(b) l'exposition de l'échantillon à la lumière, éventuellement dans lequel : (i) la lumière est choisie parmi la lumière ultraviolette et la lumière visible ; (ii) la lumière est une lumière visible provenant d'une diode électroluminescente ; ou (iii) la lumière est une lumière bioluminescente.

15. Procédé selon la revendication 14, comprenant également la mise en contact de l'échantillon avec un photocatalyseur à l'étape (a).
